(19) Europäisches Patentamt
European Patent Office
Office européen des brevets

(11) Veröffentlichungsnummer: **0 208 200**
**B1**

(12) **EUROPÄISCHE PATENTSCHRIFT**

(45) Veröffentlichungstag der Patentschrift: **25.04.90**

(51) Int. Cl.$^5$: **C 07 D 295/04,** A 61 K 31/445, C 07 D 211/14

(21) Anmeldenummer: **86108640.3**

(22) Anmeldetag: **25.06.86**

(54) **Phenylessigsäurederivate, diese Verbindungen enthaltende Arzneimittel und Verfahen zu ihrer Herstellung.**

(30) Priorität: **01.07.85 DE 3523466**

(43) Veröffentlichungstag der Anmeldung: **14.01.87 Patentblatt 87/03**

(45) Bekanntmachung des Hinweises auf die Patenterteilung: **25.04.90 Patentblatt 90/17**

(84) Benannte Vertragsstaaten: **AT BE CH DE FR GB IT LI LU NL SE**

(56) Entgegenhaltungen:
**DE-A-3 100 575**
**DE-A-3 225 155**
**DE-A-3 225 188**
**DE-A-3 347 565**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH**
**Postfach 1755**
**D-7950 Biberach (Riss) (DE)**

(72) Erfinder: **Grell, Wolfgang, Dr. Dipl.-Chem.**
**Amriswilstrasse 7**
**D-7950 Biberach 1 (DE)**
Erfinder: **Hurnaus, Rudolf, Dr. Dipl.-Chem.**
**Silcherstrasse 19**
**D-7950 Biberach 1 (DE)**
Erfinder: **Sauter, Robert, Dr. Dipl.-Chem.**
**Albert-Schweitzer-Weg 9**
**D-7958 Laupheim (DE)**
Erfinder: **Reiffen, Manfred, Dr. Dipl.-Chem.**
**Matthias-Erzberger-Strasse 40**
**D-7950 Biberach 1 (DE)**
Erfinder: **Rupprecht, Eckhard, Dr. Dipl.-Biologe**
**Riedbachstrasse 15**
**D-7950 Alendorf-Tannhausen (DE)**

**EP 0 208 200 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

In der EP—A—0.058.779 und in der EP—A—0.009.017, welche eine Kombination der DE—A—3.225.155 mit der DE—A—3.225.188 darstellt, werden Phenylessigsäurederivative beschrieben, welche eine Wirkung auf den Stoffwechsel, insbesondere jedoch eine blutzuckersenkende Wirkung, aufweisen.

Es wurde nun gefunden, daß die neuen Phenylessigsäurederivative der allgemeinen Formel

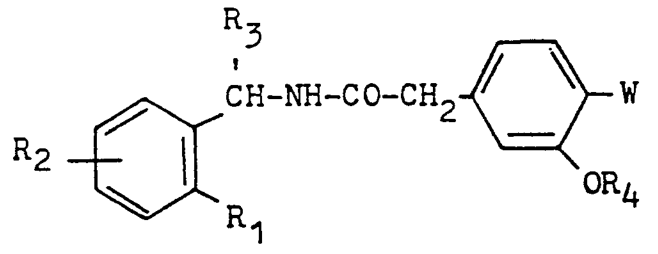

,(I)

die sich durch den Rest —$OR_4$ von den vorbeschriebenen Phenylessigsäurederivaten unterscheiden, wertvolle Eigenschaften besitzen unterscheiden, wertvolle Eigenschaften besitzen. So weisen die Verbindungen der allgemeinen Formel I, in der $R_4$ eine Alkylgruppe mit 1 bis 3 Kohlenstoffatomen oder eine Allylgruppe darstellt, überlegene pharmakologische Eigenschalften auf, insbesondere eine überlegene Wirkung auf den Stoffwechsel, vorzugsweise jedoch eine überlegene blutzuckersenkende Wirkung; die Verbindungen der allgemeinen Formel I, in der $R_4$ ein Wasserstoffatom bedeutet, stellen wertvolle Zwischenprodukte zur Herstellung der vorgenannten Verbindungen dar.

In der obigen allgemeinen Formel I bedeuten

$R_1$ eine Pyrrolidino-, Piperidino-, 4-Methyl-piperidinio-, 3-Methyl-piperidino-, 3,3-Dimethyl-piperidino-, 3,5-Dimethyl-piperidino- oder Hexamethyleniminogruppe,

$R_2$ ein Wasserstoff-, Fluor- oder Chloratom, eine Methyl- oder Methoxygruppe,

$R_4$ ein Wasserstoffatom, eine Alkylgruppe mit 1 bis 3 Kohlenstoffatomen oder eine Allylgruppe.

$R_3$ eine durch eine Tetrahydrofuranyl-, Tetrahydropyranyl- oder Cycloalkylgruppe substituierte Alkylgruppe mit 1 oder 2 Kohlenstoffatomen, in der der Cycloalkylteil 3 bis 7 Kohnlenstoffatome enthalten kann, eine Alkenylgruppe mit 3 bis 5 Kohlenstoffatomen oder eine Alkinylgruppe mit 3 oder 4 Kohnlenstoffatomen und

W eine Methyl-, Hydroxymethyl-, Formyl-, Carboxy- oder Alkoxycarbonylgruppe, wobei der Alkoxyteil 1 bis 4 Kohlenstoffatome enthalten und durch eine Phenylgruppe substituiert sein kann.

Für die bei der Definition der Reste $R_3$, $R_4$ und W eingangs erwähnten Bedeutungen kommen beispielsweise für $R_3$ die der 1-Propen-1-yl-, 2-Methyl-1-propen-1-yl-, 3-methyl-2-buten-2-yl-, 2-Propen-1-yl-, 2-Methyl-2-propen-1-yl, 2-Buten-1-yl, 2-Methyl-2-buten-1-yl, 3-methyl-2-buten-1-yl, 3-Buten-1-yl-, 2-Methyl-3-buten-1-yl, 3-Methyl-3-buten-1-yl-, 2-Hexen-1-yl, 1-Propin-1-yl, 2-Propin-1-yl-, 2-Butin-1-yl, 2-Pentin-1-yl-, Tetrahydrofuran-2-yl-methyl-, 2-(Tetrahydrofuran-2-yl)-äthyl-, tetrahydrofuran-3-yl-methyl-, Tetrahydropyran-2-yl-methyl-, 2-(Tetrahydropyran-2-yl)-äthyl-, Tetrahydropyran-3-yl-methyl-, Cyclopropylmethyl-, Cyclobutylmethyl-, Cyclopentylmethyl-, Cyclohexylmethyl-, Cycloheptylmethyl-, 2-Cyclopropyl-äthyl-, 2-Cyclobutyl-äthyl-, 2-Cyclopentyl-äthyl-, 2-Cyclohexyl-äthyl- oder 2-Cycloheptyl-äthyl-gruppe, für $R_4$ die des Wasserstoffatoms, der Methyl-, Äthyl-, n-Propyl-, Isopropyl- oder Allylgruppe und für W die der Methyl-, Hydroxymethyl, Formyl-, Carboxy-, Methoxycarbonyl-, Äthoxycarbonyl, n-Propoxycarbonyl, Isopropoxycarbonyl-, n-Butoxycarbonyl-, sec.Butoxycarbonyl-, Isobutoxycarbonyl-, tert.Butoxycarbonyl-, Benzyloxycarbonyl-, 1-Phenyläthoxycarbonyl-, 2-Phenyläthoxycarbonyl- oder 3-Phenylpropoxycarbonylgruppe in Betracht.

Bevorzugte Verbindungen der obigen allgemeinen Formel I sind diejenigen, in denen $R_1$ eine Pyrrolidino-, Piperidino-, 4-Methyl-piperidino-, 3-Methyl-piperidino-, 3,3-Dimethyl-piperidino-, 3,5-Dimethyl-piperidino- oder Hexamethyleniminogruppe, $R_2$ ein Wasserstoff-, Fluor- oder Chloratom, eine Methyl- oder Methoxygruppe, $R_4$ ein Wasserstoffatom oder eine Alkylgruppe mit 1 bis 3 Kohlenstoffatomen, $R_3$ eine Cycloalkylmethylgruppe, in der der Cycloalkylteil 3 bis 7 Kohlenstoffatome enthalten kann, eine Alkenylgruppe mit 3 bis 5 Kohlenstoffatomen, eine Propargyl-, Tetrahydrofuran-2-yl-methyl-, Tetrahydrofuran-3-yl-methyl, Tetrahydropyran-2-yl-methyl- oder Tetrahydropyran-3-yl-methylgruppe und W eine Carboxy-, Methoxycarbonyl-, Äthoxycarbonyl- oder Benzyloxycarbonylgruppe bedeuten.

Besonders bevorzugte Verbindungen der obigen allgemeinen Formel I sind jedoch diejenigen, in denen $R_1$ eine Piperidinogruppe, $R_2$ eine Wasserstoff-, Fluor- oder Chloratom, $R_4$ eine Methyl- oder Äthylgruppe, $R_3$ eine Cyclopropylmethyl-, Cyclobutylmethyl, Cyclopentylmethyl-, Cyclohexylmethyl-, Tetrahydrofuran-2-yl-methyl-, Allyl-, Methallyl-, 2-Methyl-vinyl-, 2,2-Dimethylvinyl- oder Propargylgruppe und W eine Carboxy-, Methoxycarbonyl- oder Athoxycarbonylgruppe bedeuten bedeuten, insbesondere jedoch diejenigen Verbindungen, in denen $R_1$ eine Piperidinogruppe, $R_2$ eine Wasserstoffatom, $R_4$ eine Äthylgruppe, $R_3$ eine Cyclohexylmethyl- oder Cyclopropylmethylgruppe und W eine Carboxy- oder Äthoxycarbonylgruppe bedeuten, deren Enantiomere und deren Salze, insbesondere deren physiologisch verträgliche Salze mit anorganischen oder organischen Säuren oder Basen.

Ganz besonders bevorzugte Verbindungen der obigen allgemeinen Formel I sind diejenigen, in denen W die Carboxygruppe darstellt.

Erfindungsgemäß erhält man die neuen Verbindungen nach folgenden Verfarhen:

a) Umsetzung eines Amins der allgemeinen Formel

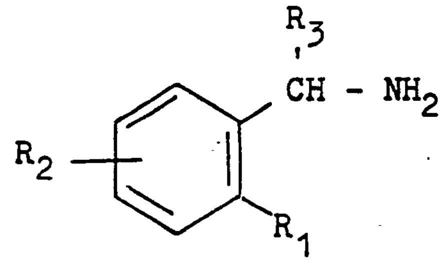

,(II)

in der

$R_1$ bis $R_3$ wie eingangs definiert sind, mit einer Carbonsäure der allgemeinen Formel

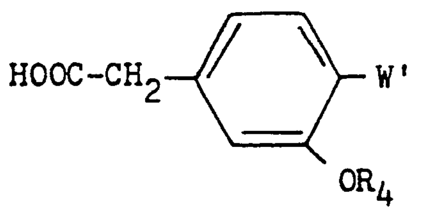

,(III)

in der

$R_4$ wie eingangs definiert ist und

W' die für W eingangs erwähnten Bedeutungen besitzt oder eine durch einen Schutzrest geschützte Carboxygruppe darstellt, oder mit deren gegebenenfalls in Reaktionsgemisch hergestellten reaktionsfähigen Derivaten und erforderlichenfalls anschließende Abspaltung eines verwendenten Schutzrestes.

Als reaktionsfähige Derivate einer Verbindung der allgemeinen Formel III kommen beispielsweise deren Ester wie der Methyl-, Äthyl- oder Benzylester, deren Thioester wie der Methylthio- oder Äthylthioester, deren Halogenide wie das Säurechlorid, deren Anhydride oder Imidazolide in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivieirenden Mittels oder eines wasserentzehenden Mittels, z.B. in Gegenwart von Chlorameisensäureäthylester, Thionylchlorid, Phosphortrichlorid, Phosphorpentoxid, N,N'-Dicyclohexylcarbodiimide, N,N'-Dicyclohexylcarbodiimid/N-Hydroxysuccinimid, N,N'-Carbonyldiimidazol oder N,N,'-Thionyldiimdazol oder Triphenylphosphin/Tetrachlorokohlenstoff, oder eines die Aminogruppe aktivierenden Mittels, z.B. Phosphortrichlorid, und gegebenenfalls in Gegennart einer anorganischen Base wie Natriumcarbonat oder einer tertiären organischen Base wie Triäthlyamin oder Pyridin, welche gleichzeitig als Lösungsmittel dienen können, bei Temperaturen zwischen −25 und 250°C, vorzugsweise jedoch bei Temperaturen zwischen −10°C und der Siedetemperatur des verwendeten Lösungsmittels, durchgeführt. Die Umsetzung kann auch ohne Lösungsmittel durchgeführt werden, desweiteren kann während Umsetzung entstehendes Wasser durch azeotrope Destillation, z.B. durch Erhitzen mit Toluol am Wasserrabscheider, oder durch Zugabe eines Trockenmittels wie Mangensiumsulfat oder Molekularsieb abgetrennt werden.

Erforderlichenfalls wird die anschließende Abspaltung eines Schutzrestes vorzugsweise hydrolytisch durchgeführt, zweckmäßgerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen −10 und 120°C, z.B. bei Temperaturen zwischen Raumtemperatur und der Siedetemperatur des Reaktionsgemisches.

Ein als Schutzrest verwendeter tert.Butylrest kann auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure abgespalten werden.

Desweiteren kann ein als Schutzrest verwendeter Benzylrest auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid abgespalten werden.

b) Zur Herstellung von Verbindungen der allgemeinen Formel I, in der W eine Carboxygruppe darstellt: Hydrolyse, Thermolyse, Hydrogenolyse oder Alkoholyse einer Verbindung der allgemeinen Formel

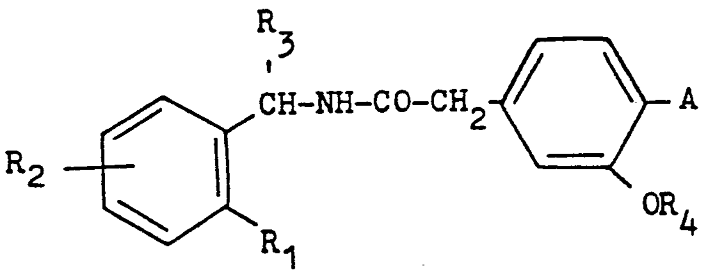

, (IV)

in der

$R_1$ bis $R_4$ wie eingangs definiert sind und

A eine durch Hydrolyse, Thermolyse oder Hydrogenolyse in eine Carboxygruppe überführbare Gruppe darstellt.

Als hydrolysierbare Gruppen kommen beispielsweise funktionell Derivate der Carboxygruppe wie deren unsubstituierte oder substituierte Amide, Ester, Thioester, Orthoester, Iminoäther, Amidine oder Anhydride, die Nitrilgruppe, die Tetrazolylgruppe, eine gegebenenfalls substituierte 1,3-Oxazol-2-yl- oder 1,3-Oxazolin-2-yl-gruppe, als thermolytisch spaltbare Gruppen beispielsweise Ester mit tertiären Alkoholen, z.B. der tert.Butylester, als hydrogenolytisch spaltbare Gruppen beispielsweise Aralkylgruppen, z.B. die Benzylgruppe, und als alkoholytisch spaltbare Gruppe die Cyanogruppe in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperturen zwischen −10 und 120°C, z.B. bei Temperaturen zwischen Raumtemperatur und der Siedetemperatur des Reaktionsgemisches, und die Alkoholyse einer Cyangruppe vorzugsweise in einem Überschuß des entsprechenden Alkohols wie Methanol, Äthanol oder Propanol und in Gegenwart einer Säure wie Chlorwasserstoff bei erhöhten Temperaturen, z.B. bei der Siedetemperatur des Reaktionsgemsiches, durchgeführt.

Bedeutet A in einer Verbindung der allgemeinen Formel IV eine Nitril- oder Aminocarbonylgruppe, so können diese Gruppen mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in eine entsprechende Carboxyverbindung übergeführt werden.

Bedeutet A in einer Verbindung der allgemeinen Formel IV beispielsweise die tert.Butyloxycarbonylgruppe, so kann die tert.Butylgruppe auch thermisch gegebenenfalls in einem inerten Lösungmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bie Temperaturen zwischen 40 und 100°C, abgespalten werden.

Bedeutet A in einer Verbindung der allgemeinen Formel IV beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur un einem Wasserstoffdruck von 1 bis 5 bar abgespalten werden. Bei der Hydrogenolyse kann gleichzeitig eine Halogenhaltige Verbindung enthalogeniert, eine vorhandene Doppel- oder Dreifachbindung aufhydriert und eine vorhandene Benzyloxycarbonylgruppe in eine Carboxygruppe übergeführt werden.

c) Zur Herstellung von Verbindungen der allgemeinen Formel I, in der $R_4$ ein Wasserstoffatom darstellt:

Abspaltung eines Schutzrestes von einer Verbindung der allgemeinen Formel

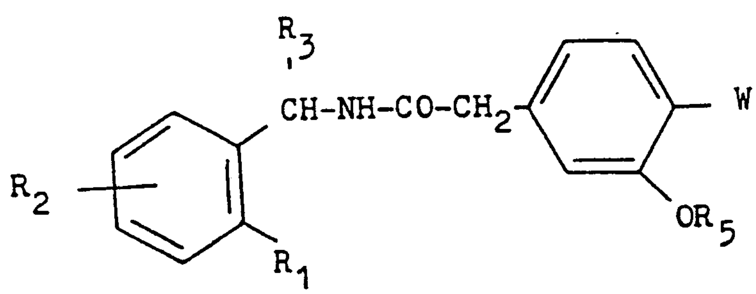

, (V)

in der

$R_1$ bis $R_3$ und W wie eingangs definiert sind und

$R_5$ eine Schutzgruppe für eine Hydroxygruppe darstellt.

Als Schutzgruppe für $R_5$ kommt beispielsweise eine Alkyl-, Aralkyl- oder Trialkylsilylgruppe, z.B. die Methyl-, Äthyl-, Propyl-, Allyl-, Benzyl- oder Trimethylsilylgruppe, in Betracht.

Die Abspaltung der oben genannten Schutzreste erfolgt je nach dem verwendeten Schutzrest entweder mittels Hydrolyse oder mittels Hydrogenolyse gegebenenfalls in einem geeigneten Lösungsmittel bei Temperaturen zwischen −78 und 250°C.

Beispielsweise erfolgt die Ätherspaltung in Gegenwart einer Säure wie Chlorwasserstoff, Bromwasserstoff, Schwefelsäure, Bortribromid, Aluminiumtrichlorid oder Pyridinhydrochlorid zweckmäßigerweise in einem geeigeneten Lösungsmittel wie Methylenchlorid, Eisessig oder Wasser oder in deren Gemischen bei Temperaturen zwischen −78 und 250°C. Hierbei wird die Ätherspaltung in Gegenwart einer Protonensäure zweckmäßigerweise bei Temperaturen zwischen 0 und 150°C, vorzugsweise bei Temperaturen zwischen 50 und 150°C, oder mit einer Lewis-Säure vorzugsweise in einem Lösungsmittel wie Methylenchlorid bei Temperaturen zwischen −78 und 20°C, durchgeführt.

Beispielsweise erfolgt die hydrogenolytische Abspaltung einer verwendeten Schutzgruppe wie der Benzylgruppe mit Wasserstoff in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid, vorzugsweise z.B. bei Raumtemperatur, und einem Wasserstoffdruck von 1 bis 5 bar.

d) Zur Herstellung von Verbindungen der allgemeinen Formel I, in der $R_4$ eine Alkylgruppe mit 1 bis 3 Kohlenstoffatomen oder eine Allylgruppe darstellt:

Umsetzung einer Verbindung der allgemeinen Formel

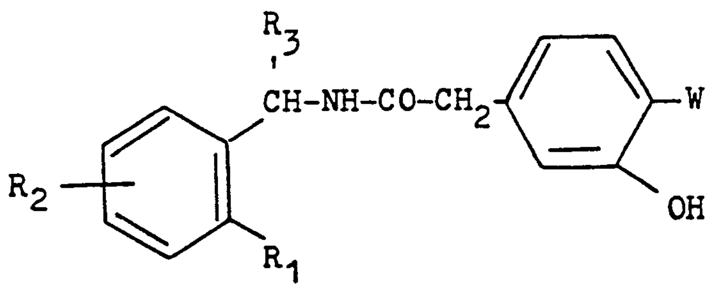

, (VI)

in der

$R_1$ bis $R_3$ und W wie eingangs definiert sind, mit einer Verbindung der allgemeinen Formel

$$X{-}R_6, \qquad (VII)$$

in der

$R_6$ eine Alkylgruppe mit 1 bis 3 Kohlenstoffatomen oder eine Allylgruppe und

X eine nukleophil austauschbare Gruppe wie ein Halogenatom, eine Sulfonyloxygruppe oder auch zusammen mit dem benachbarten Wasserstoffatom eine Diazogruppe, wenn $R_6$ eine Alkylgruppe mit 1 bis 3 Kohlenstoffatomen darstellt, bedeuten und erforderlichenfalls anschließende Hydrolyse.

Die Umsetzung wird zweckmäßigerweise mit einem entsprechenden Halogenid, Sulfonsäureester, Schwefelsäurediester oder Diazoalkan, z.B. mit Methyljodid, Dimethylsulfat, Äthylbromid, Diäthylsulfat, Propylbomid, Isopropylbromid, Allylbromid, p-Toluolsulfonsäure-äthylester, Methansulfonsäure-isopropylester oder Diazomethan, gegebenenfalls in Gegenwart einer Base wie Natriumhydrid, Kaliumcarbonat, Natriumhydroxid, Kalium-tert.butylat oder Triäthylamin in einem geeigneten Lösungsmittel wie Aceton, Diäthyläther, Tetrahydrofuran, Dioxan oder Dimethylformamid bei Temperaturen zwischen 0 und 100°C, vorzugsweise bei Temperaturen zwischen 20 und 50°C, durchgeführt.

Bedeutet in einer Verbindung der allgemeinen Formel VI W eine Carboxygruppe, so kann diese Verbindung gleichzeitig in die entsprechende Esterverbindung übergeführt werden. Eine so erhaltene Verbindung wird erforderlichenfalls durch Spaltung der Estergruppe in die gewünschte Verbindung der allgemeinen Formel I übergeführt.

Die Spaltung der Estergruppe erfolgt hydrolytisch zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Methanol, Methanol/Wasser, Äthanol, Äthanol/Wasser, Wasser/Isopropanol oder Wasser/Dioxan bei Temperaturen zwischen −10 und 120°C, z.B. bei Temperaturen zwischen Raumtemperatur und der Siedetemperatur des Reaktionsgemisches.

e) Zur Herstellung von Verbindungen der allgemeinen Formel I, in der

$R_1$ bis $R_3$ wie eingangs definiert sind,

$R_4$ ein Wasserstoffatom oder eine Alkylgruppe mit 1 bis 3 Kohlenstoffatomen und

W eine Methyl-, Formyl-, Carboxy- oder Alkoxycarbonylgruppe, wobei der Alkoxyteil 1 bis 4 Kohlenstoffatomen enthalten kann, bedeuten:

Umsetzung einer Verbindung der allgemeinen Formel

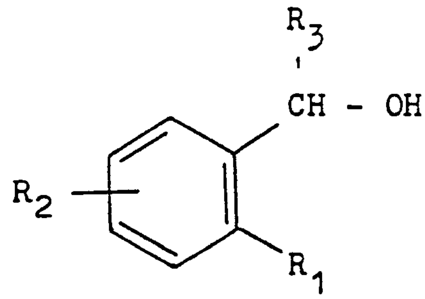

,(VIII)

in der $R_1$ bis $R_3$ wie eingangs definert sind, mit einer Verbindung der allgemeinen Formel

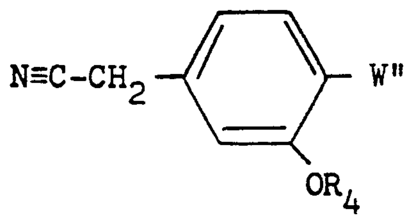

,(IX)

in der

$R_4$ wie eingangs definiert ist und

W'' eine Methyl-, Formyl-, Carboxy- oder Alkoxycarbonylgruppe, wobei der Alkoxyteil 1 bis Kohlenstoffatomen enthalten kann, darstellt.

Die Umsetzung wird in Gegenwart einer starken Säure, welche gleichzeitig als Lösungsmittel dienen kann, vorzugsweise in konzentrierter Schwefelsäure, bei Temperaturen zwischen 0 und 150°C, vorzugsweise bei Temperaturen zwischen 20 und 100°C, durchgeführt.

f) Zur Herstellung von Verbindungen der allgemeinen Formel I, in der

$R_4$ ein Wasserstoffatom oder eine Alkylgruppe mit 1 bis 3 Kohlenstoffatomen,

$R_3$ eine durch eine Tetrahydrofuranyl-, Tetrahydropyranyl- oder Cycloalkylgruppe substituierte Alkylgruppe mit 1 oder 2 Kohlenstoffatomen, in der der Cycloalkylteil 5 bis 7 Kohlenstoffatome enthalten kann, und

W eine Methyl-, Hydroxymethyl-, Carboxy- oder Alkoxycarbonylgruppe, wobei der Alkoxyteil 1 bis 4 Kohlenstoffatome enthalten kann, bedeuten;

Reduktion einer Verbindung der allgemeinen Formel

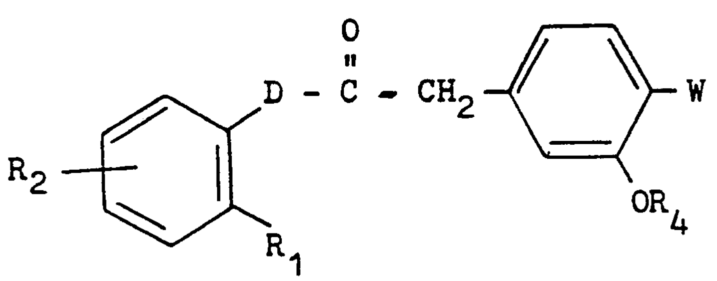

,(X)

in der

$R_1$, $R_2$, $R_4$ und W wie eingangs definiert sind und

D eine Gruppe der Formel

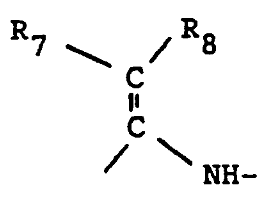

darstellt, wobei

$R_7$ und $R_8$ zusammen mit dem dazwischenliegenden Kohlenstoffatom eine durch eine Tetrahydrofuranyl-, Tetrahydropyranyl- oder Cycloalkylgruppe substituierte Alkylidengruppe mit 1 oder 2 Kohlenstoffatomen, in der Cycloalkylteil 5 bis 7 Kohlenstoffatome enthalten kann, bedeuten.

Die Reduktion wird vorzugsweise mit Wasserstoff in Gegenwart eines Hydrierungskatalysators wie

Palladium/Kohle oder Raney-Nickel in einem geeigneten Lösungsmittel wie Methanol, Äthanol, Isopropanol, Essigsäureäthylester, Dioxan, Tetrahydrofuran, Dimethylformamid, Benzol oder Benzol/Äthanol bei Temperaturen zwischen 0 und 100°C, vorzugsweise bei Temperaturen zwischen 20 und 50°C, und einem Wasserstoffdruck von 1 bis 5 bar durchgeführt. Bei Verwendung eines geeigneten chiralen Hydrierungskatalysators wie einem Metalligandenkomplex, z.B. [(2S),(4S)-tert.Butoxycarbonyl-4-diphenylphosphino-2-diphenylphosphinomethyl-pyrrolidin-rhodium-cyclooctadien(1,5)]-perchlorat, erfolgt die Wasserstoffanlagerung enantioselektiv. Desweiteren können bei der katalytischen Hydrierung andere Gruppen, z.B. eine Benzyloxygruppe zur Hyroxygruppe oder eine Formylgruppe zur Hydroxymethylgruppe, mitreduziert oder durch Wasserstoffatome, z.B. ein Halogenatom durch ein Wasserstoffatom, ersetzt werden.

Eine so erhaltene racemische Verbindung der allgemeinen Formel I kann mittels ihrer diasteromeren Addukte, Komplexe, Salze oder Derivate in die Enantiomeren aufgetrennt werden.

Die nachträgliche Racematspaltung wird vorzugsweise mittels Säulen- oder HPL-Chromatographie durch Bildung diastereomerer Addukte oder Komplexe an einer chiralen Phase durchgeführt.

Die erfindungsgemäß erhaltenen Verbindungen der allgemeinen Formel I lassen sich ferner in ihre Salze und zur pharmazeutischen Anweundung in ihre physiologisch verträglichen Salze mit anorganischen oder organischen Säuren oder auch Basen überführen. Als Säuren kommen hierbei beispielsweise Salzsäure, Bromwasserstoffsäure, Schwefelsäure, Phosphorsäure, Milchsäure, Zitronensäure, Weinsäure, Bernsteinsäure, Maleinsäure, Fumarsäure, Asparaginsäure oder Glutaminsäure und als Basen Natriumhydroxid, Kaliumhydroxid, Kalziumhydroxid, Cyclohexylamin, Äthanolamin, Diäthanolamin, Triäthanolamin, Äthylendiamin, Lysin oder Arginin in Betracht.

Die als Ausgangsstoffe verwendeten Verbindungen der allgemeinen Formeln II bis X sind teilweise literaturbekannt bzw. erhält man nach an sich bekannten Verfahren.

So erhält man beispielsweise eine Verbindung der allgemeinen Formel II durch Reduktion eines entsprechenden Nitrils mit Lithiumaluminiumhydrid oder mit katalytisch angeregtem Wasserstoff, durch Umsetzung eines entsprechenden Nitrils mit einer entsprechenden Grignard- oder Lithium-Verbindung und anschließender Lithiumaluminiumhydrid-Reduktion oder anschließender Hyroylse zum Ketimin, welches anschließend mit katalytisch angeregtem Wasserstoff, mit einem komplexen Metallhydrid oder mit nascierendem Wasserstoff reduziert wird, durch Hydrolyse bzw. durch Hydrazinolyse einer entsprechenden Phthalimidoverbindung, durch Umsetzung eines entsprechenden Ketons mit Ammoniumformiat und anschließender Hydrolyse bzw. mit einem Ammoniumsalz in Gegenwart von Natriumcyanoborhydrid, durch Reduktion eines entsprechenden Oxims mit Lithiumaluminiumhydrid, mit katalytisch angeregtem oder nascierendem Wasserstoff, durch Reduktion einer entsprechenden N-Benzyl- oder N-1-Phenyläthyl-Schniff'schen Base, z.B. mit einem komplexen Metallhydrid in Äther oder Tetrahydrofuran bei Temperaturen zwischen −78°C und der Siedetemperatur das verwendeten Lösungsmittels und anschließender Abspaltung der Benzyl- oder 1-Phenyläthylgruppe mittels katalytischer Hydrierung, durch Lithiierung einer entsprechenden Benzyldenimino-benzyl-Verbindung, z.B. mittels Lithium-diisopropylamid bei Temperaturen zwischen −78 und 20°C, anschließende Umsetzung mit einer entsprechenden Halogenverbindung, z.B. mit einer entsprechenden Bromalkyl-, Bromalkenyl- oder Bromalkinylverbindung, und anschließende Hydrolyse, durch Ritter-Reaktion eines entsprechenden Alkohols mit Kaliumcyanid in Schwefelsäure, durch Hofmman-, Curtius-, Lossen- oder Schmidt-Abbau einer entsprechenden Verbindung oder durch Überführung eines entsprechenden Benzaldehydes in ein entsprechendes Glycin-Derivat, z.B. mittel Natriumcyanid/Ammoniumcarbonat in Äthanol/Wasser in ein entsprechendes Hydantoin-Derivat, dessen Hydrolyse sowie erforderlichenfalls anschließende Veresterung und erforderlichenfalls anschließende Reduktion, z.B. mit einem komplexen Metallhydrid in Äther oder Tetrahydrofuran.

Ein so erhaltenes Amin der allgemeinen Formel II mit einem chiralen Zentrum kann durch Racematspaltung, z.B. mittels fraktionierter Kristallisation der diastereomeren Salze mit optisch akitven Säuren und anschließender Zerlegung der Salze oder durch Säulen- oder HPL-Chromatographie gegebenenfalls in Form seines Acylderivates oder durch Bildung von diastereomeren Verbindungen, deren Trennung und aschließende Spaltung in dessen Enantiomeren aufgetrennt werden.

Ferner kann ein optisch aktives Amin der allgemeinen Formel II auch durch enantio-selective Reduktion eines entsprechenden Ketimins mittels komplexer Bor- oder Aluminiumhydride, in denen Teil der Hydridwasserstoffatome durch optisch aktive Alkoholatreste ersetzt ist, oder mittels Wasserstoff in Gegenwart eines geeigneten chiralen Hydrierungskatalysators bzw. analog ausgehend von einem entsprechenden N-Benzyl- oder N-(1-Phenäthyl)-ketimin oder von einem entsprechenden N-Acyl-ketimin bzw. Enamid und gegebenenfalls anschiließende Abspaltung des Benzyl-, 1-Phenäthyl- oder Acylrestes hergestellt werden.

Ferner kann ein optisch aktives Amin der allgemeinen Formel II auch durch diastereo-selektive Reduktion eines entsprechenden am Stickstoffatom mit einem chiralen Rest substituierten Ketimins oder Hydrazons mittels komplexer oder auch nichtkomplexer Bor- oder Aluminiumhydride, in denen gegebenenfalls ein Teil der Hydriedwasserstoffe durch entsprechende Alkoholat-, Phenolat- oder auch Alkyl-Reste ersetzt ist, oder mittels Wasserstoff in Gegenwart eines geeigneten Hydrierungskatalysators und gegebenenfalls anschließende Abspaltung des chiralen Hilfsrestes durch katalytische Hydrogenolyse oder Hydrolyse hergestellt werden.

Ferner kann ein optisch aktives Amin der allgemeinen Formel II auch durch diastereo-selektive

Addition einer entsprechenden metallorganischen Verbindung, vorzugsweise einer Grignard- oder einer Lithiumverbindung, an ein entsprechendes am Stickstoffatom mit einem chiralen Rest substituiertes Aldimin, durch anschließende Hydrolyse und gegebenenfalls anschließende Abspaltung des chiralen Hilfrestes durch katalytische Hydrogenolyse oder Hydrolyse hergestellt werden.

Die als Ausgangsstoffe verwendeten Verbindungen der allgemeinen Formeln IV, V und VI erhält man durch Umsetzung eines entsprechenden Amins mit einer entsprechenden Carbonsäure bzw. deren reaktiven Derivaten und gegebenenfalls anschließende Abspaltung eines verwendeten Schutzrestes.

Eine als Ausgangstoff verwendeter Verbindung der allgemeinen Formel VIII erhält man durch Reduktion einer entsprechenden Carbonylverbindung oder durch Umstzung einer entsprechenden Carbonylverbindung mit einem entsprechenden Grignard- oder Lithiumreagens.

Eine als Ausgangsstoff verwendete Verbindung der allgemeinen Formel X erhält man durch Acylierung eines entsprechenden Ketimins oder dessen metallorganischen Komplex mit einer entsprechenden Carbonsäure oder deren reaktionsfähigen Derivaten gegebenenfalls unter Tautomerisierung.

Wie bereits eingangs erwähnt, weisen die neuen Verbindungen der allgemeinen Formel I und deren physiologisch verträglichen Salze wertvolle pharmakologische Eigenschaften auf, nämlich eine Wirkung auf den Intermediärstoffwechsel, insbesondere jedock eine blutzuckersenkende Wirkung.

Beispielsweise wurden die Verbindungen

A = 2-Äthoxy-4-[N-(α-cyclohexylmethyl-2-piperidino-benzyl)aminocarbonylmethyl]-benzoesäure-äthylester,

B = 2-Äthoxy-4-[N-(α-cyclohexylmethyl-2-piperidino-benzyl)aminocarbonylmethyl]-benzoesäure,

C = 2-Äthoxy-4-[N-(α-cyclopropylmethyl-2-piperidino-benzyl)aminocarbonylmethyl]-benzoesäure,

D = 2-Äthoxy-4-[N-(α-(tetrahydrofuran-2-yl-methyl)-2-piperidinoo-benzyl)aminocarbonylmethyl]-benzoesäure,

E = 2-Äthoxy-4-[N-(1-(2-piperidino-phenyl)-3-buten-1-yl)aminocarbonylmethyl]-benzoesäure,

F = 2-Äthoxy-4-[N-(1-(2-piperidino-phenyl)-3-methyl-3-buten-1-yl)aminocarbonylmethyl]-benzoesäure und

G = 2-Äthoxy-4-[N-(1-(2-piperidino-phenyl)-3-butin-1-yl)aminocarbonylmethyl]-benzoesäure

auf ihre blutzuckersenkende Eigenschaft wie folgt untersucht:

Blutzuckersenkende Wirkung

Die blutzuckersenkende Wirkung der zu unterschenden Substanzen wurde an weiblichen Ratten eigener Zucht mit dem Gewicht von 180—200 g geprüft, welche 24 Stunden vor Versuchsbeginn nüchtern gesetzt wurden. Die zu untersuchenden Substanzen wurden unmittelbar vor Versuchbeginn in 1,5%iger Methylcellulose suspendiert und per Schlundsonde appliziert.

Die Blutentnahme erfolgte unmittelbar vor Substanzapplikation sowie 1, 2, 3 und 4 Stunden danach, jeweils aus dem retroorbitalen Venenplexus. Hiervon wurden jeweils 50 [1 mit 0,5 ml 0,33 N Perchlorsäure enteiweßt und zentrifugiert. Im Überstand Warde Glukose nach der Hexokinase-Methode mit Hilfe eines Analysenphotometers bestimmt. Die statistische Auswertung erfolgte nach dem t-Test nach Student mit p = 0,05 als Signifikazgrenze.

Die nachfolgende Tabelle enthält die gefundenen Werte in Prozent gegenüber Kontrolle:

| Sub-stanz | 10 mg/kg | | | | 0,5 mg/kg | | | |
|---|---|---|---|---|---|---|---|---|
| | 1 | 2 | 3 | 4 h | 1 | 2 | 3 | 4 h |
| A | -16 | -14 | -17 | -16 | | | | |
| B | | | | | -22 | -47 | -45 | -45 |
| C | | | | | -45 | -45 | -36 | -36 |
| D | | | | | -46 | -25 | -13 | -10 |
| E | | | | | -42 | -39 | -28 | -35 |
| F | | | | | -44 | -41 | -31 | -28 |
| G | | | | | -33 | -18 | -11 | n.s. |

n.s. = statistisch nicht signifikant

Bei der Prüfung der Substanzen auf ihre blutzuckersenkende Wirkung konnten selbst bei einer Dosis von 10 mg/kg p.o. keine toxischen Nebenwirkungen beobachtet werden.

Die neuen Verbindungen sind praktisch untoxisch; beispielsweise verstarben nach einer einmaligen Dosis von 500 mg/kg p.o. (Suspension in 1%iger Methylcellulose) der Substanz C an jeweils 3 männliche und 3 weibliche Mäuse keines der Tiere während der Nachbeobachtungszeit von 14 Tagen.

Aufgrund ihrer pharmakologischen Eigenschaften eigen sich die erfindungsgemäß hergestellten Verbindungen der allgemeinen Formel I und deren physioligisch verträgliche Salze zer Behandlung des Diabetes mellitus. Hierzu lassen sie sich, gegebenenfalls in Kombination mit anderen Wirksubstanzen, in die üblichen galenischen Zubereitungsformen wie Tabletten, Dragées, Kapseln, Pulver oder Suspensionen einarbeiten. Die Einzeldosis am Erwachsenen beträgt hierbei 1—50 mg, vorzugsweise jedoch 2,5—20 mg, 1 oder 2 mal täglich.

Die nachfolgenden Beispiele sollen die Erfindung näher erläutern:

## Beispiel 1

2-Äthoxy-4-[N-(α-cyclohexylmethyl-2-piperidino-benzyl)-aminocarbonylmethyl]-benzosäure-äthylester

Zu einer Lösung von 1,13 g (3,96 mMol) α-Cyclohexylmethyl-2-piperidino-benzylamin in 11 ml Acetonitril gibt man nacheinander 1 g (3,96 mMol) 3-Athoxy-4-äthoxycarbonyl-phenylessigsäure, 1,25 g (4,76 mMol) Triphenylphosphin, 1,11 ml (7,92 mMol) Triäthylamin und 0,38 ml (3,96 mMol) Tetrachlorkohlenstoff und rührt 15 Stunden bei Raumtemperatur. Anschließend dampft man im Vakuum ein und verteilt zwischen Äthylacetat un Wasser. Den organischen Extrakt trocknet und filtriert man und dampft ihn im Vakumm ein. Den Eindampfrückstand reingt man durch Säulenchromatographie am Kieselgel (Toluol/Aceton = 10/1).

Ausbeute: 1,4 g (68% der Theorie),
Schmelzpunkt: 95—97°C (Petroläther/Cyclohexan = 1/1)

| | | | | | | |
|---|---|---|---|---|---|---|
| Ber.: | C | 73,81 | H | 8,52 | N | 5,38 |
| Gef.: | | 73,98 | | 8,49 | | 5,61 |

## Beispiel 2

2-Äthoxy-4-[N-(α-cyclohexylmethyl-2-piperidino-benzyl)-aminocarbonylmethyl]-benzoesäure

Man rührt 1,15 g (2,21 mMol) 2-äthoxy-4-[N-(α-cyclohexylmethyl-2-piperidino-benzyl)-aminocarbonylmethyl]-benzoesäure-äthylester in 12 ml Äthanol zusammen mit 3,3 ml 1N-Natronlauge 2 Stunden bei 50°C. Dann gibt 3,3 ml 1N-Salzsäure zu und kühlt in Eis ab. Den ausgefallenen Niederschlag filtriert man ab, wäscht ihn mit wenig eiskaltem Äthanol und trocknet ihn im Vakuum bei 100°C.

Ausbeute: 0,9 g (82% der Theorie),
Schmelzpunkt: 153—156°C

| | | | | | | |
|---|---|---|---|---|---|---|
| Ber.: | C | 73.14 | H | 8,18 | N | 5,69 |
| Gef.: | | 73,30 | | 8,17 | | 5,66 |

## Beispiel 3

2-Äthoxy-4-[N-(α-cyclopropylmethyl-2-piperidino-benzyl)-aminocarbonylmethyl]-benzoesäure-äthylester

Hergestellt analog Beispiel aus α-Cyclopropylmethyl-2-piperidino-benzylamin und 3-Äthoxy-4-äthoxycarbonylphenylessigsäure.

Ausbeute: 29,5% der Theorie,
Schmelzpunkt: 126—127°C

| | | | | | | |
|---|---|---|---|---|---|---|
| Ber.: | C | 72,77 | H | 8,00 | N | 5,85 |
| Gef.: | | 72,85 | | 7,74 | | 5,84 |

## Beispiel 4

2-Äthoxy-4-[N-(α-cyclopropylmethyl-2-piperidino-benzyl)-aminocarbonylmethyl]-benzoesäure-semihydrat

Hergestellt analog Beispiel 2 durch alkalische Verseifung von 2-Äthoxy-4-[N-(α-cyclopropylmethyl-2-piperidino-benzyl)-aminocarbonylmethyl]-benzoesäureäthylester.

Ausbeute: 88% der Theorie,
Schmelzpunkt: 103—104°C

| | | | | | | |
|---|---|---|---|---|---|---|
| Ber. (× 0,5 $H_2O$): | C | 70,55 | H | 7,68 | N | 6,10 |
| Gef.: | | 70,67 | | 7,67 | | 6,37 |

## Beispiel 5

2-Äthoxy-4-[N-(α-cyclobutylmethyl-2-piperidino-benzyl)-aminocarbonylmethyl]-benzoesäure-äthylester

Hergestellt analog Beispiel 1 aus α-Cyclobutylmethy-2-piperidino-benzylamin und 3-Äthoxy-4-äthoxycarbonyl-phenyl-essigsäure.

Ausbeute: 14% der Theorie,
Schmelzpunkt: 116—118°C

| | | | | | | |
|---|---|---|---|---|---|---|
| Ber.: | C | 73,14 | H | 8,18 | N | 5,69 |
| Gef.: | | 73,14 | | 8,32 | | 5,64 |

Beispiel 6

2-Äthoxy-4-[N-(α-cyclobutylmethyl-2-piperidino-benzyl)-aminocarbonylmethyl]-benzoesäure

Hergestellt analog Beispiel 2 durch alkalische Verseifung von 2-Äthoxy-4-[N-(α-cyclobutylmethyl-2-piperidino-benzyl)-aminocarbonylmethyl]-benzoesäure-äthylester.

Ausbeute: 33% der Theorie,
Schmelzpunkt: 140—142°C

| | | | | | | |
|---|---|---|---|---|---|---|
| Ber.: | C | 72,39 | H | 7,81 | N | 6,03 |
| Gef.: | | 72,15 | | 7,79 | | 5,97 |

Beispiel 7

2-Äthoxy-4-[N-(α-cyclopentylmethyl-2-piperidino-benzyl)-aminocarbonylmethyl]-benzoesäure-äthylester

Hergestellt analog Beispiel 1 aus α-Cyclopentylmethyl-2-piperidino-benzylamin und 3-Äthoxy-4-äthoxycarbonyl-phenyl-essigsäure.

Ausbeute: 36,8% der Theorie,
Schmelzpunkt: 120—121°C

| | | | | | | |
|---|---|---|---|---|---|---|
| Ber.: | C | 73,49 | H | 8,36 | N | 5,53 |
| Gef.: | | 73,31 | | 8,55 | | 5,39 |

Beispiel 8

2-Äthoxy-4-[N-(α-cyclopentylmethyl-2-piperidino-benzyl)-aminocarbonylmethyl]-benzoesäure

Hergestellt analog Beispiel 2 durch alkalische Verseifung von 2-Äthoxy-4-[N-(α-cyclopentylmethyl-2-piperidino-benzyl)-aminocarbonylmethyl]-benzoesäure-äthylester.

Ausbeute: 75% der Theorie,
Schmelzpunkt: 85—88°C

| | | | | | | |
|---|---|---|---|---|---|---|
| Ber.: | C | 72,77 | H | 8,00 | N | 5,85 |
| Gef.: | | 72,50 | | 8,02 | | 6,03 |

Beispiel 9

2-Äthoxy-4-[N-(2-piperidino-α-(tetrahydrofuran-2-yl-methyl)-benzyl)-aminocarbonylmethyl]-benzoesäure-äthylester

Hergestellt analog Beispiel 1 aus 2-Piperidino-α-(tetrahydrofuran-2-yl-methyl)-benzylamin und 3-Äthoxy-4-äthoxycarbonyl-phenylessigsäure.

Ausbeute: 38% der Theorie,
Schmelzpunkt: 111—113°C

| | | | | | | |
|---|---|---|---|---|---|---|
| Ber.: | C | 70,84 | H | 7,93 | N | 5,51 |
| Gef.: | | 70,76 | | 7,73 | | 5,51 |

Beispiel 10

2-Äthoxy-4-[N-(2-piperidino-α-(tetrahydrofuran-2-yl-methyl)-benzyl)-aminocarbonylmethyl]-benzoesäure

Hergestellt analog Beispiel 2 durch alkalische Verseifung von 2-Äthoxy-4-[N-(2-piperidino-α-(tetrahydrofuran-2-yl-methyl)-benzyl)-aminocarbonylmethyl]-benzoesäure-äthylester.

Ausbeute: 75% der Theorie,
Schmelzpunkt: 121—123°C

| | | | | | | |
|---|---|---|---|---|---|---|
| Ber.: | C | 69,98 | H | 7,55 | N | 5,83 |
| Gef.: | | 69,90 | | 7,78 | | 5,71 |

Beispiel 11

2-Äthoxy-4-[N-(α-cycloheptylmethyl-2-piperidino-benzyl)-aminocarbonylmethyl]-benzoesäure-äthylester

Hergestellt analog Beispiel 1 aus α-Cyclopentylmethylbenzylamin und 3-Äthoxy-4-äthoxycarbonyl-phenylessigsäure.

Ausbeute: 48% der Theorie,
Schmelzpunkt: 96—98°C

| | | | | | | |
|---|---|---|---|---|---|---|
| Ber.: | C | 74,12 | H | 8,67 | N | 5,24 |
| Gef.: | | 74,40 | | 8,87 | | 5,39 |

Beispiel 12

2-Äthoxy-4-[N-(α-cycloheptylmethyl-2-piperidino-benzyl)-aminocarbonylmethyl]-benzoesäure

Hergestellt analog Beispiel 2 durch alkalische Verseifung von 2-Äthoxy-4-[N-(α-cycloheptylmethyl-2-piperidino-benzyl)-aminocarbonylmethyl]-benzoesäure-äthylester.

Ausbeute: 83% der Theorie,
Schmelzpunkt: 127—130°C

| | | | | | | |
|---|---|---|---|---|---|---|
| Ber.: | C | 73,49 | H | 8,36 | N | 5,53 |
| Gef.: | | 73,54 | | 8,62 | | 5,47 |

Beispiel 13

2-Äthoxy-4-[N-(α-cyclohexylmethyl-2-piperidino-benzyl)-aminocarbonylmethyl]-benzoesäure-äthylester

a) 2-Äthoxy-4-[N-(α-(cyclohexyl-methyliden)-2-piperidino-benzyl)-aminocarbonylmethyl]-benzoesäure-äthylester

Hergestellt analog Beispiel 1 aus α-Cyclohexylmethyl-α'-(2-piperidino-phenyl)-ketimin und 3-Äthoxy-4-äthoxycarbonyl-phenylessigsäure.

Ausbeute: 25% der Theorie,
Schmelzpunkt: 85—88°C

| | | | | | | |
|---|---|---|---|---|---|---|
| Ber.: | C | 74,10 | H | 8,16 | N | 5,10 |
| Gef.: | | 74,37 | | 8,00 | | 5,45 |

Laut 80 MHz-$^1$H-NMR-Spektrum ($CDCl_3$) liegt ein Gemisch E/Z = 1/2 vor. [Olefinisches H: (E) D 6,26, (Z) 5,42 ppm].

b) 2-Äthoxy-4-[N-(α-cyclohexylmethyl-2-piperidino-benzyl)-aminocarbonylmethyl]-benzoesäure-äthylester

Hergestellt durch katalytische Hydrierung von 2-Äthoxy-4-[N-(α-(cyclohexyl-methyliden)-2-piperidino-benzyl)-aminocarbonylmethyl]-benzoesäure-äthylester an Palladium/Kohle (10%ig) in Äthanol bei Raumtemperatur und 3 bar Wasserstoff.

Ausbeute: 61% der Theorie,
Schmelzpunkt: 95—97°C

| | | | | | | |
|---|---|---|---|---|---|---|
| Ber.: | C | 73,81 | H | 8,52 | N | 5,38 |
| Gef.: | | 73,92 | | 8,74 | | 5,29 |

Beispiel 14

2-Äthoxy-4-[N-(α-cyclohexylmethyl-2-piperidino-benzyl)-aminocarbonylmethyl]-benzoesäure

a) 2-Äthoxy-4-[N-(α-(cyclohexyl-methyliden)-2-piperidino-benzyl)-aminocarbonylmethyl]-benzoesäure

Hergestellt analog Beispiel 2 durch alkalische Verseifung von 2-Äthoxy-4-[N-(α-(cyclohexyl-methyliden)-2-piperidino-benzyl)-aminocarbonylmethyl]-benzoesäure-äthylester.

Ausbeute: 87% der Theorie,
Schmelzpunkt: 95—100°C

| | | | | | | |
|---|---|---|---|---|---|---|
| Ber.: | C | 73,44 | H | 7,81 | N | 5,71 |
| Gef.: | | 73,38 | | 7,73 | | 5,75 |

b) 2-Äthoxy-4-[N-(α-cyclohexylmethyl-2-piperidino-benzyl)-aminocarbonylmethyl]-benzoesäure

Hergestellt analog Beispiel 13 durch katalytische Hydrierung von 2-Äthoxy-4-[N-(α-(cyclohexyl-methyliden)-2-piperidino-benzyl)-aminocarbonylmethyl]-benzoesäure.

Ausbeute: 52% der Theorie,
Schmelzpunkt: 154—156°C

| | | | | | | |
|---|---|---|---|---|---|---|
| Ber.: | C | 73,14 | H | 8,18 | N | 5,69 |
| Gef.: | | 73,31 | | 8,25 | | 5,71 |

Beispiel 15

2-Äthoxy-4-[N-(1-(2-piperidino-phenyl)-3-buten-1-yl)-aminocarbonylmethyl]-benzoesäure-äthylester

Hergestellt analog Beispiel 1 aus 1-(2-Piperidino-phenyl)-3-buten-1-yl-amin und 3-Äthoxy-4-äthoxycarbonyl-phenylessigsäure.

Ausbeute: 32,7% der Theorie,
Schmelzpunkt: 110—112°C

| | | | | | | |
|---|---|---|---|---|---|---|
| Ber.: | C | 72,39 | H | 7,81 | N | 6,03 |
| Gef.: | | 72,10 | | 7,66 | | 5,94 |

Beispiel 16

2-Äthoxy-4-[N-(1-(2-piperidino-phenyl)-3-buten-1-yl)-aminocarbonylmethyl]-benzoesäure

Hergestellt analog Beispiel 2 durch alkalische Verseifung von 2-Äthoxy-4-[N-(1-(2-piperidino-phenyl)-3-buten-1-yl)-aminocarbonylmethyl]benzosäure-äthylester.

Ausbeute: 68% der Theorie,
Schmelzpunkt: 92—95°C

| | | | | | | |
|---|---|---|---|---|---|---|
| Ber.: | C | 71,53 | H | 7,39 | N | 6,42 |
| Gef.: | | 71,27 | | 7,42 | | 6,42 |

Beispiel 17

2-Äthoxy-4-[N-(1-(2-piperidino-phenyl)-3-methyl-3-buten-1-yl)-aminocarbonylmethyl]-benzoesäure-äthylester

Herestellt analog Beispiel 1 aus 3-Methyl-1-(2-piperidino-phenyl)-3-buten-1-yl)-amin und 3-Äthoxy-4-äthoxycarbonyl-phenylessigsäure.

Ausbeute: 33,6% der Theorie,
Schmelzpunkt: 126—128°C

| | | | |
|---|---|---|---|
| Ber.: | C 72,77 | H 8,00 | N 5,85 |
| Gef.: | 72,82 | 8,22 | 5,78 |

Beispiel 18

2-Äthoxy-4-[N-(1-(2-piperidino-phenyl)-3-methyl-3-buten-1-yl)-aminocarbonylmethyl]-benzoesäure

Hergestellt analog Beispiel 2 durch alkalische Verseifung von 2-Äthoxy-4-[N-(1-(2-piperidino-phenyl)-3-methyl-3-buten-1-yl)-aminocarbonylmethyl]-benzoesäure-äthylester.

Ausbeute: 74% der Theorie,
Schmelzpunkt: 64—66°C

| | | | |
|---|---|---|---|
| Ber.: | C 71,97 | H 7,61 | N 6,22 |
| Gef.: | 71,70 | 7,50 | 5,98 |

Beispiel 19

2-Äthoxy-4-[N-(1-(2-piperidino-phenyl)-3-methyl-3-buten-1-yl)-aminocarbonylmethyl]-benzoesäure-äthylester
[mit einem 25%igen Anteil an 2-Äthoxy-4-[N-(1-(2-piperidino-phenyl)-3-methyl-1-butyl)-aminocarbonylmethyl]-benzoesäure-äthylester]

Hergestellt analog Beispiel 1 aus 3-Methyl-1-(2-piperidino-phenyl)-2-buten-1-yl-amin [mit einem 25%igen Anteil an 3-Methyl-1-(2-piperidino-phenyl)-1-butylamin] und 3-Äthoxy-4-äthoxycarbonyl-phenylessigsäure.

Ausbeute: 37% der Theorie,
Schmelzpunkt: 141—142°C

| | | | |
|---|---|---|---|
| Ber.: | C 72,77 | H 8,00 | N 5,85 |
| Gef.: | 72,60 | 7,77 | 5,73 |

Das Mischungsverhältnis 75/25 ergibt sich aus dem entsprechenden Verhältnis der Intensitäten der besonders charakteristischen Signale im 400 MHz-$^1$H-NMR-Spektrum ($CDCl_3$).

Die Lage der Signale ist: 3-Methyl-2-buten-1-yl-Verbindung: olefinisches H: 5,25 (d), $CH_3$: 1.64 (s) und 1,77 (s), benzylische >CH— 6,00 (t), benzylisches —$CH_2$—: 3,52 ppm (s).

3-Methyl-1-butyl-Verbindung: $CH_3$: 0,90 (d), benzylisches >CH— 5,35 (m), benzylisches —$CH_2$—: 3,54 ppm (s).

Beispiel 20

2-Äthoxy-4-[N-(1-(2-piperidino-phenyl)-3-methyl-2-buten-1-yl)-aminocarbonylmethyl]-benzoesäure
[mit einem 25%igen Anteil an 2-Äthoxy-4-[N-(1-(2-piperidino-phenyl)-3-methyl-1-butyl)-aminocarbonylmethyl]-benzoesäure]

Hergestellt analog Beispiel 2 durch alkalische Verseifung des entsprechenden Äthylester-Gemisches aus Beispiel 19.

Ausbeute: 91% der Theorie,
Schmelzpunkt: 154—156°C

| | | | |
|---|---|---|---|
| Ber.: | C 71,97 | H 7,61 | N 6,22 |
| Gef.: | 71,80 | 7,57 | 5,98 |

Beispiel 21

2-Äthoxy-4-[N-(1-(2-piperidino-phenyl)-3-butin-1-yl)-aminocarbonylmethyl]-benzoesäure-äthylester

Hergestellt analog Beispiel 1 aus 1-(2-Piperidino-phenyl)-3-butin-1-yl-amin und 3-Äthoxy-4-äthoxycarbonyl-phenylessigsäure.

Ausbeute: 52% der Theorie,
Schmelzpunkt: 86—90°C

| | | | |
|---|---|---|---|
| Ber.: | C 72,70 | H 7,41 | N 6.06 |
| Gef.: | 72,60 | 7,40 | 6.04 |

Beispiel 22

2-Äthoxy-4-[N-(1-(2-piperidino-phenyl)-3-butin-1-yl)-aminocarbonylmethyl]-benzoesäure

Hergestellt analog Beispiel 2 durch alkalische Verseifung von 2-Äthoxy-4-[N-(1-(2-piperidino-phenyl)-3-butin-1-yl)-aminocarbonylmethyl]-benzoesäure-äthylester.

Ausbeute: 68% der Theorie,
Schmelzpunkt: 66—69°C

| | | | |
|---|---|---|---|
| Ber.: | C 71,87 | H 6,96 | N 6,45 |
| Gef.: | 71,60 | 6,95 | 6,38 |

Beispiel 23

2-Äthoxy-4-[N-(1-(2-piperidino-phenyl)-4-penten-1-yl)-aminocarbonylmethyl]-benzoesäure-äthylester

Hergestellt analog Beispiel 1 aus 1-(2-Piperidino-phenyl)-4-penten-1-yl-amin und 3-Äthoxy-4-äthoxycarbonyl-phenylssigsäure.

Ausbeute: 58% der Theorie,
Schmelzpunkt: 117—120°C

| | | | |
|---|---|---|---|
| Ber.: | C 72,77 | H 8,00 | N 5,85 |
| Gef.: | 72,73 | 7,97 | 6.07 |

Beispiel 24

2-Äthoxy-4-[N-(1-(2-piperidino-phenyl)-4-penten-1-yl)-aminocarbonylmethyl]-benzoesäure

Hergestellt analog Beispiel 2 durch alkalische Verseifung von 2-Äthoxy-4-[N-(1-(2-piperidino-phenyl)-4-penten-1-yl)-aminocarbonylmethyl]-benzoesäure-äthylester.

Ausbeute: 61% der Theorie,
Schmelzpunkt: 82—85°C

| | | | |
|---|---|---|---|
| Ber.: | C 71,97 | H 7,61 | N 6,22 |
| Gef.: | 71,97 | 7,59 | 5,98 |

Beispiel 25

2-Äthoxy-4-[N-(1-(2-piperidino-α-(tetrahydropyran-2-yl-methyl)-benzyl)-aminocarbonylmethyl]-benzoesäure-äthylester

Hergestellt analog Beispiel 1 aus 2-Piperidino-α-(tetrahydropyran-2-yl-methyl)-benzylamin und 3-Äthoxy-4-äthoxycarbonyl-phenylessigsäure.

Ausbeute: 29% der Theorie,
Schmelzpunkt: 82—85°C

| | | | |
|---|---|---|---|
| Ber.: | C 71,24 | H 8,10 | N 5,36 |
| Gef.: | 71,28 | 7,96 | 5,29 |

Beispiel 26

2-Äthoxy-4-[N-(1-(2-piperidino-α-(tetrahydropyran-2-yl-methyl)-benzyl)-aminocarbonylmethyl]-benzoesäure

Hergestellt analog Beispiel 2 durch alkalische Verseifung von 2-Äthoxy-4-[N-(1-(2-piperidino-α-(tetrahydropyran-2-yl-methyl)-benzyl)-aminocarbonylmethyl]-benzoesäure-äthylester.

Ausbeute: 85% der Theorie,
Schmelzpunkt: 140—142°C (sintern ab 70°C; Teilerweichung bei 105°C)

| | | | |
|---|---|---|---|
| Ber.: | C 70,42 | H 7,74 | N 5,66 |
| Gef.: | 70,15 | 7,88 | 5,40 |

Beispiel 27

2-Äthoxy-4-[N-(α-cyclohexylmethyl-2-piperidino-benzyl)-aminocarbonylmethyl]-benzoesäure-äthylester

Zu einem Gemisch von 15 ml konzentrierter Schwefelsäure und 15 ml o-Dichlorbenzol tropft man bei 23—25°C eine Lösung von 2,35 g (10 mMol) 2-Äthoxy-4-cyanomethyl-benzoesäure-äthylester und 2,88 g (10 mMol) α-Cyclohexylmethyl-2-piperidino-benzylalkohol in 15 ml o-Dichlorbenzol. Man rührt 2 Stunden bei Raumtemperatur nach. Anschließend trennt man die o-Dichlorbenzol-Phase ab und gibt den Rückstand auf Eis. Nach Alkalisieren mit Sodalösung wird mit Chloroform extrahiert. Die Extrakte werden über Natriumsulfat getrocknet und eingeengt. Der Rückstand wird durch Säulenchromatographie an Kieselgel (Toluol/Aceton = 10/1) gereinigt.

Ausbeute: 1,1 g (21% der Theorie),
Schmelzpunkt: 95—97°C

| | | | |
|---|---|---|---|
| Ber.: | C 73,81 | H 8,52 | N 5,38 |
| Gef.: | 73,95 | 8,64 | 5,42 |

Analog den vorstehenden Beispielen werden folgende Verbindungen erhalten:

2-Äthoxy-4-[N-(α-cyclopropylmethyl-2-pyrrolidino-benzyl)-aminocarbonylmethyl]-benzoesäure-äthylester

2-Äthoxy-4-[N-(α-cyclopropylmethyl-2-pyrrolidino-benzyl)-aminocarbonylmethyl]-benzoesäure

2-Äthoxy-4-[N-(α-cyclopropylmethyl-2-hexamethylenimino-benzyl)-aminocarbonylmethyl]-benzoesäure-äthylester

2-Äthoxy-4-[N-(α-cyclopropylmethyl-2-hexamethylenimino-benzyl)-aminocarbonylmethyl]-benzoesäure

2-Äthoxy-4-[N-(α-cyclopropylmethyl-2-(4-methyl-piperidino)-benzyl)-aminocarbonylmethyl]-benzoesäure-äthylester

2-Äthoxy-4-[N-(α-cyclopropylmethyl-2-(4-methyl-piperidino)-benzyl)-aminocarbonylmethyl]-benzoesäure

4-[N-(α-Cyclcopropylmethyl-2-piperidino-benzyl)-aminocarbonylmethyl]-2-methoxy-benzoesäure-äthylester
4-[N-(α-Cyclcopropylmethyl-2-piperidino-benzyl)-aminocarbonylmethyl]-2-methoxy-benzoesäure
2-Äthoxy-4-[N-(α-cyclohexylmethyl-2-pyrrolidino-benzyl)-aminocarbonylmethyl]-benzoesäure-äthylester
2-Äthoxy-4-[N-(α-cyclohexylmethyl-2-pyrrolidino-benzyl)-aminocarbonylmethyl]-benzoesäure
2-Äthoxy-4-[N-(α-cyclohexylmethyl-2-hexamethylenimino-benzyl)-aminocarbonylmethyl]-benzoesäure-äthylester
2-Äthoxy-4-[N-(α-cyclohexylmethyl-2-hexamethylenimino-benzyl)-aminocarbonylmethyl]-benzoesäure
2-Äthoxy-4-[N-(α-cyclohexylmethyl-2-(4-methyl-piperidino)-benzyl)-aminocarbonylmethyl]-benzoesäure-äthylester
2-Äthoxy-4-[N-(α-cyclohexylmethyl-2-(4-methyl-piperidino)-benzyl)-aminocarbonylmethyl]-benzoesäure
2-Äthoxy-4-[N-(α-cyclohexylmethyl-2-(3,5-dimethyl-piperidino)-benzyl)-aminocarbonylmethyl]-benzoesäure-äthylester
2-Äthoxy-4-[N-(α-cyclohexylmethyl-2-(3,5-dimethyl-piperidino)-benzyl)-aminocarbonylmethyl]-benzoesäure
2-Äthoxy-4-[N-(α-cyclohexylmethyl-2-(3,3-dimethyl-piperidino)-benzyl)-aminocarbonylmethyl]-benzoesäure-äthylester
2-Äthoxy-4-[N-(α-cyclohexylmethyl-2-(3,3-dimethyl-piperidino)-benzyl)-aminocarbonylmethyl]-benzoesäure
4-[N-(α-Cyclohexylmethyl-2-piperidino-benzyl)-aminocarbonylmethyl]-2-methoxy-benzoesäure-äthylester
4-[N-(α-Cyclohexylmethyl-2-piperidino-benzyl)-aminocarbonylmethyl]-2-hydroxy-benzoesäure
4-[N-(α-Cyclohexylmethyl-2-piperidino-benzyl)-aminocarbonylmethyl]-2-methoxy-benzoesäure-äthylester
4-[N-(α-Cyclohexylmethyl-2-piperidino-benzyl)-aminocarbonylmethyl]-2-methoxy-benzoesäure
2-Allyloxy-4-[N-(α-cyclohexylmethyl-2-piperidino-benzyl)-aminocarbonylmethyl]-benzoesäure-äthylester
2-Allyloxy-4-[N-(α-cyclohexylmethyl-2-piperidino-benzyl)-aminocarbonylmethyl]-benzoesäure
4-[N-(α-Cyclohexylmethyl-2-piperidino-benzyl)-aminocarbonylmethyl]-2-n-propopxy-benzoesäure-äthylester
4-[N-(α-Cyclohexylmethyl-2-piperidino-benzyl)-aminocarbonylmethyl]-2-n-propoxy-benzoesäure
2-Äthoxy-4-[N-(α-cyclohexylmethyl-4-fluor-2-piperidinobenzyl)-aminocarbonylmethyl]-benzoesäure-äthylester
2-Äthoxy-4-[N-(α-cyclohexylmethyl-4-fluor-2-piperidinobenzyl)-aminocarbonylmethyl]-benzoesäure
2-Äthoxy-4-[N-(α-cyclohexylmethyl-3-chlor-2-piperidinobenzyl)-aminocarbonylmethyl]-benzoesäure-äthylester
2-Äthoxy-4-[N-(α-cyclohexylmethyl-3-chlor-2-piperidinobenzyl)-aminocarbonylmethyl]-benzoesäure
2-Äthoxy-4-[N-(α-cyclohexylmethyl-6-chlor-2-piperidinobenzyl)-aminocarbonylmethyl]-benzoesäure-äthylester
2-Äthoxy-4-[N-(α-cyclohexylmethyl-6-chlor-2-piperidino-benzyl)-aminocarbonylmethyl]-benzoesäure
2-Äthoxy-4-[N-(α-cyclohexylmethyl-3-methyl-2-piperidinobenzyl)-aminocarbonylmethyl]-benzoesäure-äthylester
2-Äthoxy-4-[N-(α-cyclohexylmethyl-3-methyl-2-piperidinobenzyl)-aminocarbonylmethyl]-benzoesäure
2-Äthoxy-4-[N-(α-cyclohexylmethyl-4-methyl-2-piperidinobenzyl)-aminocarbonylmethyl]-benzoesäure-äthylester
2-Äthoxy-4-[N-(α-cyclohexylmethyl-4-methyl-2-piperidinobenzyl)-aminocarbonylmethyl]-benzoesäure
2-Äthoxy-4-[N-(α-cyclohexylmethyl-6-methyl-2-piperidinobenzyl)-aminocarbonylmethyl]-benzoesäure-äthylester
2-Äthoxy-4-[N-(α-cyclohexylmethyl-6-methyl-2-piperidinobenzyl)-aminocarbonylmethyl]-benzoesäure
2-Äthoxy-4-[N-(α-cyclohexylmethyl-4-methoxy-2-piperidinobenzyl)-aminocarbonylmethyl]-benzoesäure-äthylester
2-Äthoxy-4-[N-(α-cyclohexylmethyl-4-methoxy-2-piperidinobenzyl)-aminocarbonylmethyl]-benzoesäure
2-Äthoxy-4-[N-(α-cyclohexyl-äthyl)-2-piperidino-benzyl)-aminocarbonylmethyl]-benzoesäure-äthylester
2-Äthoxy-4-[N-(α-(2-cyclohexyl-äthyl)-2-piperidino-benzyl)-aminocarbonylmethyl]-benzoesäure
2-Äthoxy-4-[N-(1-(2-piperidino-phenyl)-2-buten-1-yl)-aminocarbonylmethyl]-benzoesäure-äthylester
2-Äthoxy-4-[N-(1-(2-piperidino-phenyl)-2-buten-1-yl)-aminocarbonylmethyl]-benzoesäure
2-Äthoxy-4-[N-(1-(2-piperidino-phenyl)-4-methyl-3-penten-1-yl)-aminocarbonylmethyl]-benzoesäure-äthylester
2-Äthoxy-4-[N-(1-(2-piperidino-phenyl)-4-methyl-3-penten-1-yl)-aminocarbonylmethyl]-benzoesäure

2-Äthoxy-4-[N-(1-(2-piperidino-phenyl)--3-penten-1-yl)-aminocarbonylmethyl]-benzoesäure-äthylester
2-Äthoxy-4-[N-(1-(2-piperidino-phenyl)-3-penten-1-yl)-aminocarbonylmethyl]-benzoesäure

Beispiel A

Tabletten mit 5 mg 2-Äthoxy-4-[N-(α-cyclohexylmethyl-2-piperidino-benzyl)-aminocarbonylmethyl]-benzoesäure

Zusammensetzung:

1 Tablette enthält:

| | |
|---|---|
| Wirksubstanz | (1) 5,0 mg |
| Maisstärke | (2) 62,0 mg |
| Milchzucker | (3) 48,0 mg |
| Polyvinylpyrrolidon | (4) 4,0 mg |
| Magnesiumstearat | (5) 1,0 mg |
| | 120,0 mg |

Herstellungsverfahren:

1, 2, 3 und 4 werden gemischt und mit Wasser befeuchtet. Die feuchte Mischung wird durch ein Sieb mit 1,5 mm Maschenweite gedrückt und bei ca. 45°C getrocknet. Das trockene Granulat wird durch ein Sieb mit 1,0 mm Maschenweite geschlagen und mit 5 vermischt. Die fertige Mischung preßt man auf einer Tablettenpresse mit Stempeln von 7 mm Durchmesser, die mit einer Teilkerbe versehen sind, zu Tabletten.

Tablettengewicht: 120 mg

Beispiel B

Dragées mit 2,5 mg 2-Äthoxy-4-[N-(α-cyclohexylmethyl-2-piperidino-benzyl)-aminocarbonylmethyl]-benzoesäure

1 Dragéekern enthält:

| | |
|---|---|
| Wirksubstanz | (1) 2,5 mg |
| Kartoffelstärke | (2) 44,0 mg |
| Milchzucker | (3) 30,0 mg |
| Polyvinylpyrrolidon | (4) 3,0 mg |
| Magnesiumstearat | (5) 0,5 mg |
| | 80,0 mg |

Herstellungsverfahren:

1, 2, 3 und 4 werden gemischt und mit Wasser befeuchtet. Die feuchte Masse drückt man durch ein Sieb mit 1 mm Maschenweite, trocknet bei ca. 45°C und schlägt das Granulat anschließend durch dasselbe Sieb. Nach dem Zumischen von 5 werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.

Dragéegewicht: 120 mg

Beispiel C

Tabletten mit 10 mg 2-Äthoxy-4-[N-(α-cyclohexylmethyl-2-piperidino-benzyl)-aminocarbonylmethyl]-benzoesäure

Zusammensetzung:

1 Tablette enthält:

| | |
|---|---|
| Wirksubstanz | 10,0 mg |
| Milchzucker pulv. | 70,0 mg |
| Maisstärke | 31,0 mg |
| Polyvinylpyrrolidon | 8,0 mg |
| Magnesiumstearat | 1,0 mg |
| | 120,0 mg |

Herstellungsverfahren:

Die Mischung aus der Wirksubstanz, Milchzucker und Maisstärke wird mit einer 20 %igen Lösung von Polyvinylpyrrolidon in Wasser befeuchtet. Die feuchte Masse wird durch ein Sieb mit 1,5 mm Maschenweite granuliert und bei 45°C getrocknet. Das getrocknete Granulat wird durch ein Sieb mit 1 mm Maschenweite getrieben und mit Magnesiumstearat homogen vermischt.

Tablettengewicht: 120 mg
Stempel: 7 mm Durchmesser mit Teilkerbe

Beispiel D

Dragées mit 5 mg 2-Äthoxy-4-[N-(α-cyclohexylmethyl-2-piperidino-benzyl)-aminocarbonylmethyl]-benzoesäure

1 Dragéekern enthält:

| | |
|---|---|
| Wirksubstanz | 5,0 mg |
| Calciumphosphat sekundär | 70,0 mg |
| Maisstärke | 50,0 mg |
| Polyvinylpyrrolidon | 4,0 mg |
| Magnesiumstearat | 1,0 mg |
| | 130,0 mg |

Herstellungsverfahren:

Die Mischung aus der Wirksubstanz, Calciumphosphat und Maisstärke wird mit einer 15 %igen Lösung von Polyvinylpyrrolidon in Wasser befeuchtet. Die feuchte Masse wird durch ein Sieb mit 1 mm Maschenweite geschlagen, bei 45°C getrocknet und anschließend durch dasselbe Sieb gerieben. Nach dem Vermischen mit der angegebenen Menge Magnesiumstearat werden daraus Dragéekerne gepreßt.
Kerngewicht: 130 mg
Stempel: 7 mm Durchmesser

Auf die so hergestellten Dragéekerne wird auf bekannte Art eine Schicht aus Zucker und Talkum aufgetragen. Die fertigen Dragées werden mit Wachs poliert.

Dragéegewicht: 180 mg

**Patentansprüche**

1. Phenylessigsäurederivate der allgemeinen Formel

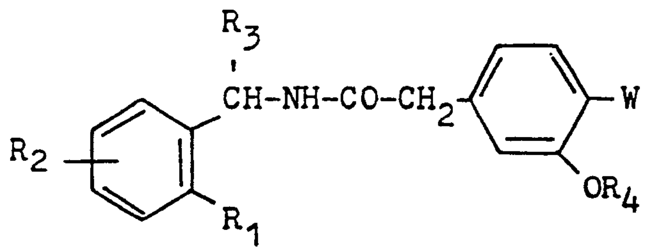 ,(I)

in der

$R_1$ eine Pyrrolidino-, Piperidino-, 4-Methyl-piperidino-, 3-Methyl-piperidino-, 3,3-Dimethyl-piperidino-, 3,5-Dimethyl-piperidino- oder Hexamethyleniminogruppe,

$R_2$ ein Wasserstoff-, Fluor- oder Chloratom, eine Methyl- oder Methoxygruppe,

$R_4$ ein Wasserstoffatom, eine Alkylgruppe mit 1 bis 3 Kohlenstoffatomen oder eine Allylgruppe,

$R_3$ eine durch eine Tetrahydrofuranyl-, Tetrahydropyranyl- oder Cycloalkylgruppe substituierte Alkylgruppe mit 1 oder 2 Kohlenstoffatomen, in der der Cycloalkylteil 3 bis 7 Kohlenstoffatome enthalten kann, eine Alkenylgruppe mit 3 bis 5 Kohlenstoffatomen oder eine Alkinylgruppe mit 3 oder 4 Kohlenstoffatomen und

W eine Methyl-, Hydroxymethyl-, Formyl-, Carboxy- oder Alkoxycarbonylgruppe, wobei der Alkoxyteil 1 bis 4 Kohlenstoffatome enthalten und durch eine Phenylgruppe substituiert sein kann, darstellen, deren Enantiomere und deren Salze.

2. Phenylessigsäurederivate der allgemeinen Formel I gemäß Anspruch 1, in der $R_1$ eine Pyrrolidino-, Piperidino-, 4-Methyl-piperidino-, 3-Methyl-piperidino-, 3,3-Dimethyl-piperidino-, 3,5-Dimethyl-piperidino- oder Hexamethyleniminogruppe, $R_2$ ein Wasserstoff-, Fluor- oder Chloratom, eine Methyl- oder Methoxygruppe, $R_4$ ein Wasserstoffatom oder eine Alkylgruppe mit 1 bis 3 Kohlenstoffatomen, $R_3$ eine Cycloalkylmethylgruppe, in der der Cycloalkylteil 3 bis 7 Kohlenstoffatome enthalten kann, eine Alkenylgruppe mit 3 bis 5 Kohlenstoffatomen, eine Propargyl-, Tetrahydrofuran-2-yl-methyl-, Tetrahydrofuran-3-yl-methyl-, Tetrahydropyran-2-yl-methyl- oder Tetrahydropyran-3-yl-methylgruppe und W eine Carboxy-, Methoxycarbonyl-, Äthoxycarbonyl- oder Benzyloxycarbonylgruppe bedeuten, deren Enantiomere und deren Salze.

3. Phenylessigsäurederivate der allgemeinen Formel I gemäß Anspruch 1, in der $R_1$ eine Piperidinogruppe, $R_2$ ein Wasserstoff-, Fluor- oder Chloratom, $R_4$ eine Methyl- oder Äthylgruppe, $R_3$ eine Cyclopropylmethyl-, Cyclobutylmethyl-, Cyclopentylmethyl-, Cyclohexylmethyl-, Tetrahydrofuran-2-yl-methyl-, Allyl-, Methallyl-, 2-Methyl-vinyl-, 2,2-Dimethylvinyl- oder Propargylgruppe und W eine Carboxy-, Methoxycarbonyl- oder Äthoxycarbonylgruppe bedeuten, deren Enantiomere und deren Salze.

4. Phenylessigsäurederivate der allgemeinen Formel I gemäß Anspruch 1, in der $R_1$ eine Piperidinogruppe, $R_2$ ein Wasserstoffatom, $R_4$ eine Äthylgruppe, $R_3$ eine Cyclohexylmethyl- oder Cyclopropylmethylgruppe und W eine Carboxy- oder Äthoxycarbonylgruppe bedeuten, deren Enantiomere und deren Salze.

5. Phenylessigsäurederivate der allgemeinen Formel I gemäß Anspruch 1, in der $R_1$ bis $R_4$ wie in den Ansprüchen 1 bis 4 definiert sind und W eine Carboxygruppe darstellt, deren Enantiomere und deren Salze.

6. 2-Äthoxy-4-[N-(α-cyclohexylmethyl-2-piperidino-benzyl)-aminocarbonylmethyl]-benzoesäure, deren Enantiomere und deren Salze.

7. Physiologisch verträgliche Salze der Verbindungen gemäß den Ansprüchen 1 bis 6 mit anorganischen oder organischen Säuren oder auch Basen.

8. Arzneimittel, enthaltend eine Verbindung gemäß den Ansprüchen 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung gemäß den Ansprüchen 1 bis 6 oder deren physiologisch verträglichen Salze gemäß Anspruch 7 zur Herstellung eines Arzneimittels, welches zur Behandlung des Diabetes mellitus geeignet ist.

10. Verfahren zur Herstellung eines Arzneimittels gemäß Anspruch 8, dadurch gekennzeichnet, daß eine Verbindung gemäß den Ansprüchen 1 bis 6 oder ein physiologisch verträgliches Salz gemäß Anspruch 7 in einen oder mehrere inerte Trägerstoffe und/oder Verdünnungsmittel eingearbeitet wird.

11. Analogieverfahren zur Herstellung der Phenylessigsäure-derivate gemäß den Ansprüchen 1 bis 7, dadurch gekennzeichnet, daß

a) ein Amin der allgemeinen Formel

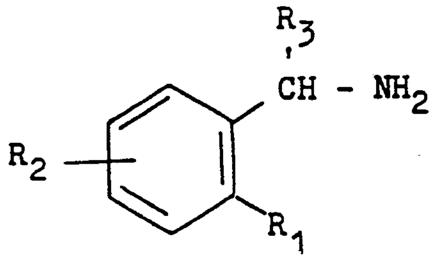

,(II)

in der

$R_1$ bis $R_3$ wie in den Ansprüchen 1 bis 6 definiert sind, mit einer Carbonsäure der allgemeinen Formel

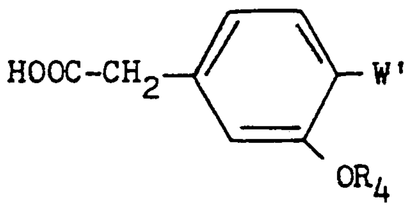

,(III)

in der

$R_4$ wie in den Ansprüchen 1 bis 6 definiert ist und

W' die für W in den Ansprüchen 1 bis 6 erwähnten Bedeutungen besitzt oder eine durch einen Schutzrest geschützte Carboxygruppe darstellt,

oder mit deren gegebenenfalls im Reaktionsgemisch hergestellten reaktionsfähigen Derivaten umgesetzt und erforderlichenfalls anschließend ein verwendeter Schutzrest abgespalten wird, oder

b) zur Herstellung von Verbindungen der allgemeinen Formel I, in der W eine Carboxygruppe darstellt, eine Verbindung der allgemeinen Formel

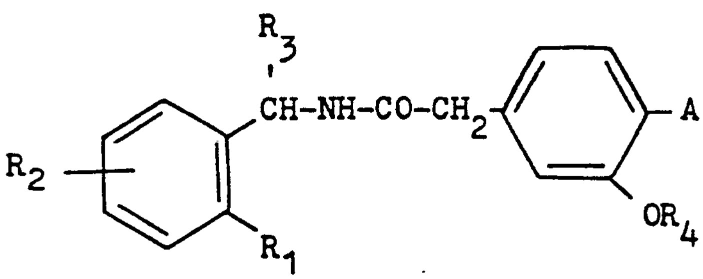

,(IV)

in der

$R_1$ bis $R_4$ wie in den Ansprüchen 1 bis 6 definiert sind und

A eine durch Hydrolyse, Thermolyse oder Hydrogenolyse in eine Carboxygruppe überführbare Gruppe darstellt, mittels Hydrolyse, Thermolyse, Hydrogenolyse oder Alkoholyse in eine entsprechende Verbindung übergeführt wird oder

c) zur Herstellung von Verbindungen der allgemeinen Formel I, in der $R_4$ ein Wasserstoffatom darstellt, ein Schutzrest von einer Verbindung der allgemeinen Formel

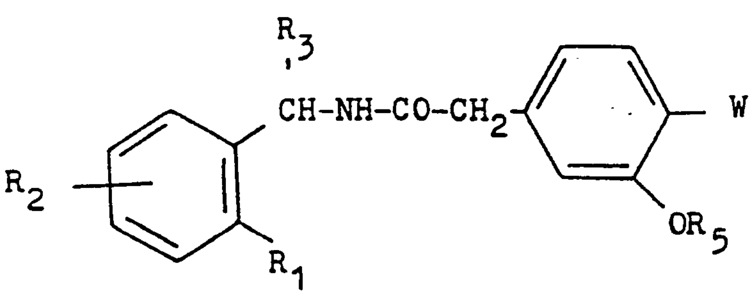

,(V)

in der

$R_1$ bis $R_3$ und W wie in den Ansprüchen 1 bis 6 definiert sind und

$R_5$ eine Schutzgruppe für eine Hydroxygruppe darstellt, abgespalten wird, oder

d) zur Herstellung von Verbindungen der allgemeinen Formel I, in der $R_4$ eine Alkylgruppe mit 1 bis 3 Kohlenstoffatomen oder eine Allylgruppe darstellt, eine Verbindung der allgemeinen Formel

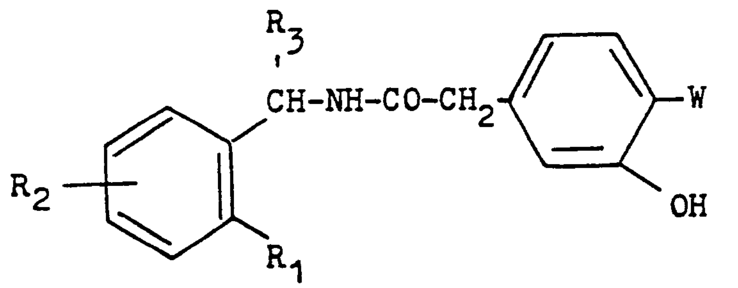

,(VI)

in der

$R_1$ bis $R_3$ und W wie in den Ansprüchen 1 bis 6 definiert sind, mit einer Verbindung der allgemeinen Formel

$$X—R_6 \quad (VII)$$

in der

$R_6$ eine Alkylgruppe mit 1 bis 3 Kohlenstoffatomen oder eine Allylgruppe und

X eine nukleophil austauschbare Gruppe wie ein Halogenatom, eine Sulfonyloxygruppe oder auch zusammen mit dem benachbarten Wasserstoffatom eine Diazogruppe, wenn $R_6$ eine Alkylgruppe mit 1 bis 3 Kohlenstoffatomen darstellt, bedeuten, umgesetzt und erforderlichenfalls eine so erhaltene Verbindung anschließend hydrolysiert wird, oder

e) zur Herstellung von Verbindungen der allgemeinen Formel I, in der

$R_1$, $R_2$ und $R_3$ wie in den Ansprüchen 1 bis 6 definiert sind,

$R_4$ ein Wasserstoffatom oder eine Alkylgruppe mit 1 bis 3 Kohlenstoffatomen und

W eine Methyl-, Formyl-, Carboxy- oder Alkoxycarbonylgruppe, wobei der Alkoxyteil 1 bis 4 Kohlenstoffatomen enthalten kann, bedeuten, eine Verbindung der allgemeinen Formel

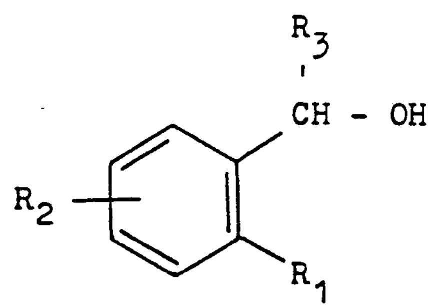

,(VIII)

in der

$R_1$ bis $R_3$ wie in den Ansprüchen 1 bis 6 definiert sind, mit einer Verbindung der allgemeinen Formel

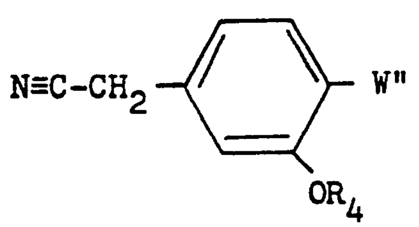

,(IX)

in der

$R_4$ wie in den Ansprüchen 1 bis 6 definiert ist und

W'' eine Methyl-, Formyl-, Carboxy- oder Alkoxycarbonylgruppe, wobei der Alkoxyteil 1 bis 4 Kohlenstoffatomen enthalten kann, darstellt, umgesetzt wird, oder

f) zur Herstellung von Verbindungen der allgemeinen Formel I, in der

$R_4$ ein Wasserstoffatom oder eine Alkylgruppe mit 1 bis 3 Kohlenstoffatomen,

$R_3$ eine durch eine Tetrahydrofuranyl-, Tetrahydropyranyl- oder Cycloalkylgruppe substituierte Alkylgruppe mit 1 oder 2 Kohlenstoffatomen, in der der Cycloalkylteil 5 bis 7 Kohlenstoffatome enthalten kann, und

W eine Methyl-, Hydroxymethyl-, Carboxy- oder Alkoxycarbonylgruppe, wobei der Alkoxyteil 1 bis 4 Kohlenstoffatome enthalten kann, bedeuten, eine Verbindung der allgemeinen Formel

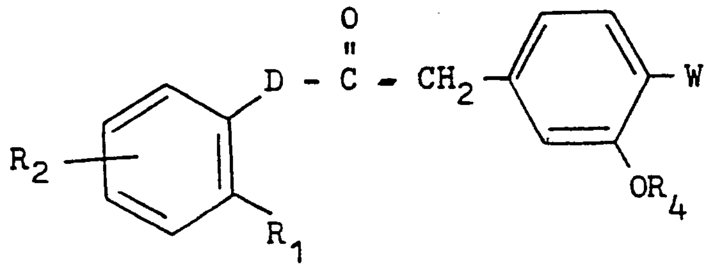

, (X)

in der

$R_1$, $R_2$, $R_4$ und W wie in den Ansprüchen 1 bis 6 definiert sind und

D eine Gruppe der Formel

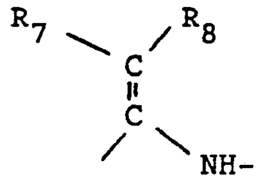

darstellt, wobei

$R_7$ und $R_8$ zusammen mit dem dazwischenliegenden Kohlenstoffatom eine durch eine Tetrahydrofuranyl-, Tetrahydropyranyl- oder Cycloalkylgruppe substituierte Alkylidengruppe mit 1 oder 2 Kohlenstoffatomen, in der Cycloalkylteil 5 bis 7 Kohlenstoffatome enthalten kann, reduziert wird, und gewünschtenfalls anschließend eine so erhaltene racemische Verbindung der allgemeinen Formel I durch Chromatographie an chiralen Phasen in ihre Enantiomeren aufgetrennt wird, und/oder eine so erhaltene Verbindung der allgemeinen Formel I in ihre Salze, insbesondere in ihre physiologisch verträglichen Salze mit anorganischen oder organischen Säuren oder Basen übergeführt wird.

**Revendications**

1. Dérivés de l'acide phénylacétique de formule générale

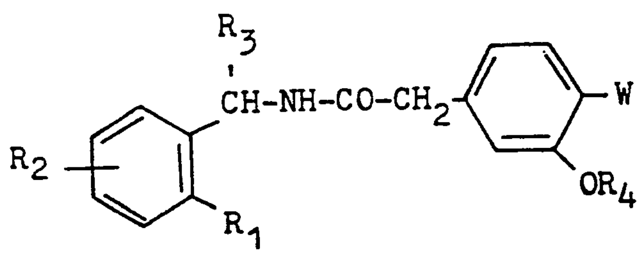

, (I)

dans laquelle

$R_1$ représente un groupe pyrrolidino, pipéridino, 4-méthyl-pipéridino, 3-méthyl-pipéridino, 3,3-diméthyl-pipéridino, 3,5-diméthyl-pipéridino ou hexaméthylènimino,

$R_2$ représente un atome d'hydrogène, de fluor ou de chlore, un groupe méthyle ou méthoxy,

$R_4$ représente un atome d'hydrogène, un groupe alcoyle avec 1 à 3 atomes de carbone ou un groupe allyle,

$R_3$ représente un groupe alcoyle avec 1 ou 2 atomes de carbone, substitué par un groupe tétrahydrofurannyle, tétrahydropyrannyle ou cycloalcoyle, dans lequel la partie cycloalcoyle peut contenir 3 à 7 atomes de carbone, ou représente un groupe alcényle avec 3 à 5 atomes de carbone ou un groupe alcynyle avec 3 ou 4 atomes de carbones et

W représente un groupe méthyle, hydroxyméthyle, formyle, carboxy ou alcoxycarbonyle, la partie alcoxy pouvant contenir 1 à 4 atomes de carbone et être substituée par un groupe phényle, leurs énantiomères et leurs sels.

2. Dérivés de l'acide phénylacétique de formule générale I selon la revendication 1, dans laquelle $R_1$ représente un groupe pyrrolidino, pipéridino, 4-méthyl-pipéridino, 3-méthyl-pipéridino, 3,3-diméthyl-pipéridino, 3,5-diméthyl-pipéridino ou hexaméthylénimino, $R_2$ représente un atome d'hydrogène, de fluor ou de chlore, un groupe méthyle ou méthoxy, $R_4$ représente un atome d'hydrogène ou un groupe alcoyle avec 1 à 3 atomes de carbone, $R_3$ représente un groupe cycloalcoylméthyle dans lequel la partie cycloalcoyle peut contenir 3 à 7 atomes de carbone, ou représente un groupe alcényle avec 3 à 5 atomes de carbone, un groupe propargyle, tétrahydrofuranne-2-yl-méthyle, tétrahydrofuranne-3-yl-méthyle, tétrahydropyranne-2-yl-méthyle ou tétrahydropyranne-3-yl-méthyle et W représente un groupe carboxy, méthoxycarbonyle, éthoxycarbonyle ou benzyloxycarbonyle, leurs énantiomères et leurs sels.

3. Dérivés de l'acide phénylacétique de formule générale I selon la revendication 1, dans laquelle $R_1$ représente un groupe pipéridino, $R_2$ représente un atome d'hydrogène, de fluor ou de chlore, $R_4$ représente un groupe méthyle ou éthyle, $R_3$ représente un groupe cyclopropylméthyle, cyclobutylméthyle, cyclopentylméthyle, cyclohexylméthyle, tétrahydrofuranne-2-yl-méthyle, allyle, méthallyle, 2-méthylvinyle, 2,2-diméthylvinyle ou propargyle et W représente un groupe carboxy, méthoxycarbonyle ou éthoxycarbonyle, leurs énantiomères et leurs sels.

4. Dérivés de l'acide phénylacétique de formule générale I selon la revendication 1, dans laquelle $R_1$ représente un groupe pipéridino, $R_2$ représente un atome d'hydrogène, $R_4$ représente un groupe éthyle, $R_3$ représente un groupe cyclohexylméthyle ou cyclopropylméthyle et
W représente un groupe carboxy ou éthoxycarbonyle, leurs énantiomères et leurs sels.

5. Dérivés de l'acide phénylacétique de formule générale I selon la revendication 1, dans laquelle $R_1$ à $R_4$ sont définis comme dans les revendications 1 à 4 et W représente un groupe carboxy, leurs énantiomères et leurs sels.

6. L'acide 2-éthoxy-4-[N-(α-cyclohexylméthyl-2-pipéridino-benzyl)-aminocarbonylméthyl]-benzoïque, ses énantiomères et ses sels.

7. Sels physiologiquement supportables des composés selon les revendications 1 à 6 avec des acides ou également des bases non organiques ou organiques.

8. Médicaments contenant un composé selon les revendications 1 à 6 ou un sel physiologiquement supportable selon la revendication 7, éventuellement conjointement à un ou plusieurs excipients et/ou diluants inertes.

9. Utilisation d'un composé selon les revendications 1 à 6 ou de ses sels physiologiquement supportables selon la revendication 7 pour la fabrication d'un médicament qui convient au traitement du diabète mellitus.

10. Procédé pour la fabrication d'un médicament selon la revendication 8, caractérisé en ce qu'on incorpore un composé selon les revendications 1 à 6 ou un sel physiologiquement supportable selon la revendication 7 dans un ou plusieurs excipients et/ou diluants inertes.

11. Procédé par analogie pour la préparation des dérivés d'acide phénylacétique selon les revendications 1 à 7, caractérisé en ce que

a) on fait réagir une amine de formule générale

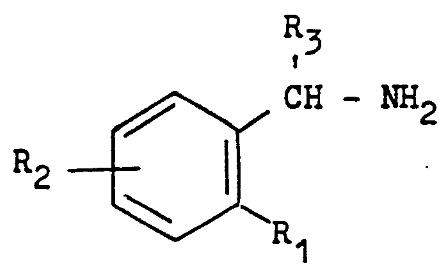

,(II)

dans laquelle
$R_1$ à $R_3$ sont définis comme dans les revendications 1 à 6, avec un acide carboxylique de formule générale

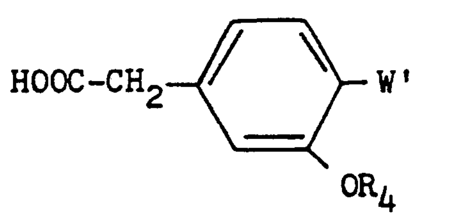

,(III)

dans laquelle
$R_4$ est défini comme dans les revendications 1 à 6 et W' possède les significations mentionnées dans les revendications 1 à 6 pour W ou représente un groupe carboxy protégé par un groupe protecteur, ou avec leurs dérivés aptes à réagir, éventuellement préparés dans le mélange réactionnel et si nécessaire, on clive ensuite un radical protecteur utilisé, ou

b) pour la préparation des composés de formule générale I dans laquelle W représente un groupe carboxy, on transforme un composé de formule générale

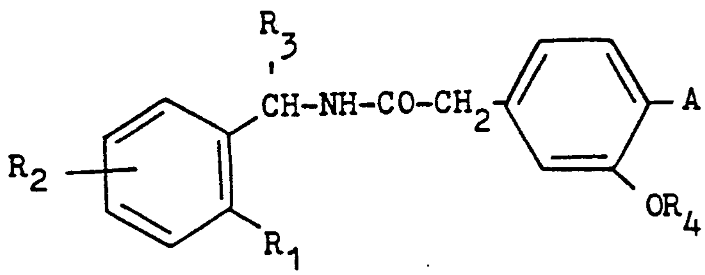

, (IV)

dans laquelle

$R_1$ à $R_4$ sont définis comme dans les revendications 1 à 6 et

A représente un groupe transformable en un groupe carboxy par hydrolyse, thermolyse ou hydrogénolyse, au moyen d'une hydrolyse, thermolyse, hydrogénolyse ou alcoolyse en un composé correspondant ou

c) pour la préparation du composés de formule générale I dans laquelle $R_4$ représente un atome d'hydrogène, on clive un radical protecteur à partir d'un composé de formule générale

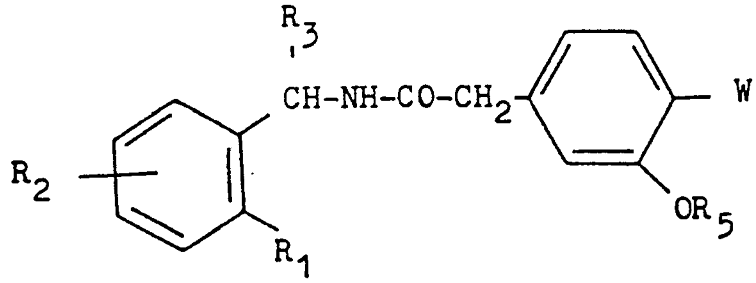

, (V)

dans laquelle

$R_1$ à $R_3$ et W sont définis comme dans les revendications 1 à 6 et

$R_5$ représente un groupe protecteur pour un groupe hydroxy, ou

d) pour la préparation des composés de formule générale I dans laquelle $R_4$ représente un groupe alcoyle avec 1 à 3 atomes de carbone ou un groupe allyle, on fait réagir un composé de formule générale

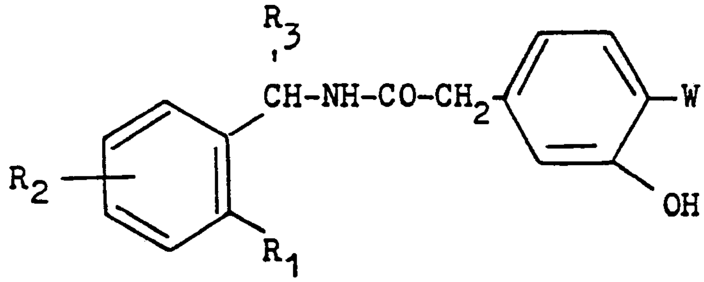

, (VI)

dans laquelle

$R_1$ à $R_3$ et W sont définis comme dans les revendications 1 à 6, avec un composé de formule générale

$$X—R_6 \qquad (VII)$$

dans laquelle

$R_6$ représente un groupe alcoyle avec 1 à 3 atomes de carbone ou un groupe allyle et

X représente un groupe échangeable de façon nucléophile tel qu'un atome d'halogène, un groupe sulfonyloxy ou également ensemble avec l'atome d'hydrogène voisin, un groupe diazo, lorsque $R_6$ représente un groupe alcoyle avec 1 à 3 atomes de carbone et si nécessaire, on hydrolyse ensuite un composé ainsi obtenu, ou

e) pour la préparation des composés de formule générale I dans laquelle

$R_1$, $R_2$ et $R_3$ sont définis comme dans les revendications 1 à 6,

$R_4$ représente un atome d'hydrogène ou un groupe alcoyle avec 1 à 3 atomes de carbone, et

W représente un groupe méthyle, formyle, carboxy ou alcoxycarbonyle, la partie alcoxy pouvant contenir 1 à 4 atomes de carbone, on fait réagir un composé de formule générale

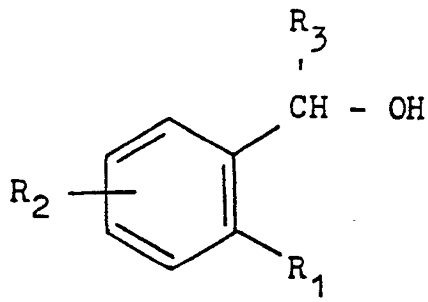

,(VIII)

dans laquelle

$R_1$ à $R_3$ sont définis comme dans les revendications 1 à 6, avec un composé de formule générale

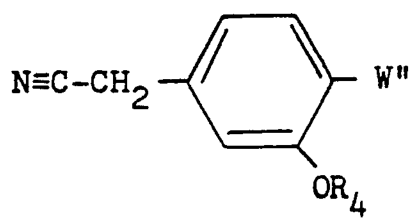

,(IX)

dans laquelle

$R_4$ est défini comme dans les revendications 1 à 6 et

W'' représente un groupe méthyle, formyle, carboxy ou alcoxycarbonyle, la partie alcoxy pouvant contenir 1 à 4 atomes de carbone, ou

f) pour la préparation des composés de formule générale I dans laquelle

$R_4$ représente un atome d'hydrogène ou un groupe alcoyle avec 1 à 3 atomes de carbone

$R_3$ représente un groupe alcoyle avec 1 à 2 atomes de carbone, substitué par tétrahydrofurannyle, tétrahydropyrannyle ou cycloalcoyle, dans lequel la partie cycloalcoyle peut contenir 5 à 7 atomes de carbone et

W représente un groupe méthyle, hydroxyméthyle, carboxy ou alcoxycarbonyle, le partie alcoxy pouvant contenir 1 à 4 atomes de carbone, on réduit un composé de formule générale

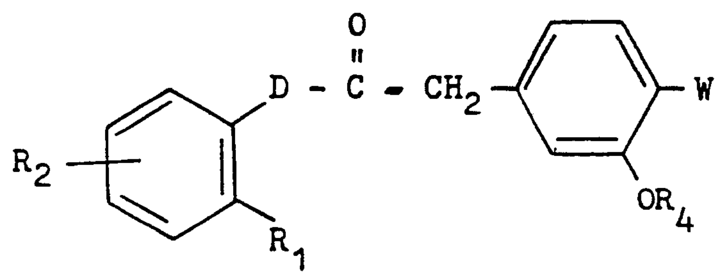

,(X)

dans laquelle

$R_1$, $R_2$, $R_4$ et W sont définis comme dans les revendications 1 à 6 et

D représente un groupe de formule

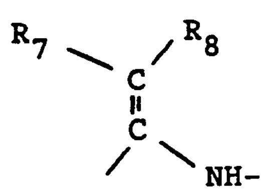

où

$R_7$ et $R_8$, ensemble avec l'atome de carbone intermédiaire représentent un groupe alcoylidène avec 1 ou 2 atomes de carbone, substitué par un groupe tétrahydrofurannyle, tétrahydropyrannyle ou cycloalcoyle, dans lequel la partie cycloalcoyle peut contenir 5 à 7 atomes de carbone, et si on le désire, on sépare ensuite un composé racémique ainsi obtenu de formule générale I par chromatographie sur phases chirales en ses énantiomères et/ou on transforme un composé ainsi obtenu de formule générale I en ses sels, en particulier en ses sels physiologiquement supportables avec des acides ou des bases non organiques ou organiques.

## Claims

1. Phenylacetic acid derivatives of general formula

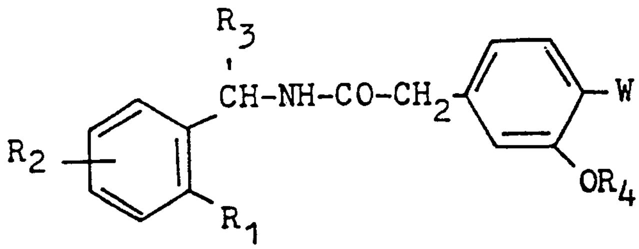

,(I)

wherein

$R_1$ represents a pyrrolidino, piperidino, 4-methylpiperidino, 3-methyl-piperidino, 3,3-dimethyl-piperidino, 3,5-dimethyl-piperidino or hexamethyleneimino group,

$R_2$ represents a hydrogen, fluorine or chlorine atom or a methyl or methoxy group,

$R_4$ represents a hydrogen atom, an alkyl group with 1 to 3 carbon atoms or an allyl group,

$R_3$ represents an alkyl group with 1 or 2 carbon atoms substituted by a tetrahydrofuranyl, tetrahydropyranyl or cycloalkyl group, in which the cycloalkyl part may contain 3 to 7 carbon atoms, an alkenyl group with 3 to 5 carbon atoms or an alkynyl group with 3 or 4 carbon atoms, and

W represents methyl, hydroxymethyl, formyl, carboxy or alkoxycarbonyl group, whilst the alkoxy part may contain from 1 to 4 carbon atoms and may be substituted by a phenyl group, and the enantiomers and salts thereof.

2. Phenylacetic acid derivatives of general formula I as claimed in claim 1, wherein $R_1$ represents a pyrrolidino, piperidino, 4-methylpiperidino, 3-methyl-piperidino, 3,3-dimethyl-piperidino, 3,5-dimethyl-piperidino or hexamethyleneimino group, $R_2$ represents a hydrogen, fluorine or chlorine atom or a methyl or methoxy group, $R_4$ represents a hydrogen atom or an alkyl group with 1 to 3 carbon atoms $R_3$ represents a cycloalkylmethyl group in which the cycloalkyl part may contain from 3 to 7 carbon atoms, an alkenyl group with 3 to 5 carbon atoms, a propargyl, tetrahydrofuran-2-yl-methyl, tetrahydrofuran-3-yl-methyl, tetrahydropyran-2-yl-methyl or tetrahydropyran-3-yl-methyl group and W represents a carboxy, methoxycarbonyl, ethoxycarbonyl or benzyloxycarbonyl group, the enantiomers and salts thereof.

3. Phenylacetic acid derivatives of general formula I as claimed in claim 1, wherein $R_1$ represents a piperidino group, $R_2$ represents a hydrogen, fluorine or chlorine atom, $R_4$ represents a methyl or ethyl group, $R_3$ represents a cyclopropylmethyl, cyclobutylmethyl, cyclopentylmethyl, cyclohexylmethyl, tetrahydrofuran-2-yl-methyl, allyl, methallyl, 2-methyl-vinyl, 2,2-dimethyl-vinyl or propargyl group and W represents a carboxy, methoxycarbonyl or ethoxycarbonyl group, the enantiomers and the salts thereof.

4. Phenylacetic acid derivatives of general formula I as claimed in claim 1, wherein $R_1$ represents a piperidino group, $R_2$ represents a hydrogen atom, $R_4$ represents an ethyl group, $R_3$ represents a cyclohexylmethyl or cyclopropylmethyl group, and W represents a carboxy or ethoxycarbonyl group, the enantiomers and salts thereof.

5. Phenylacetic acid derivatives of general formula I as claimed in claim 1, wherein $R_1$ to $R_4$ are defined as in claims 1 to 4 and W represents a carboxy group, the enantiomers and the salts thereof.

6. 2-Ethoxy-4-[N-(α-cyclohexylmethyl-2-piperidinobenzyl)-aminocarbonylmethyl]-benzoic acid, the enantiomers and the salts thereof.

7. Physiologically acceptable salts of the compounds as claimed in claim 1 to 6 with inorganic or organic acids or bases.

8. Pharmaceutical compositions containing a compound as claimed in claims 1 to 6 or a physiologically acceptable salt as claimed in claim 7 optionally together with one or more inert vehicles and/or diluents.

9. Use of a compound as claimed in claims 1 to 6 or the physiologically acceptable salts thereof as claimed in claim 7 for preparing a pharmaceutical composition suitable for the treatment of Diabetes mellitus.

10. Process for preparing a pharmaceutical composition as claimed in claim 8, characterised in that a compound as claimed in claims 1 to 6 or a physiologically acceptable salt thereof as claimed in claim 7 is incorporated in one or more inert vehicles and/or diluents.

11. Analogous process for preparing the phenylacetic acid derivatives as claimed in claims 1 to 7, characterised in that

a) an amine of general formula

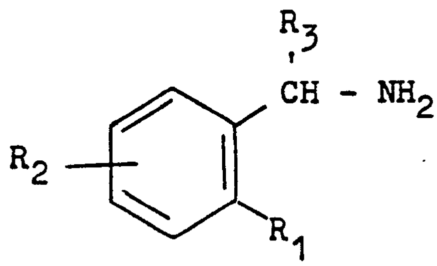

,(II)

wherein

$R_1$ to $R_3$ are defined as in claims 1 to 6, is reacted with a carboxylic acid of general formula

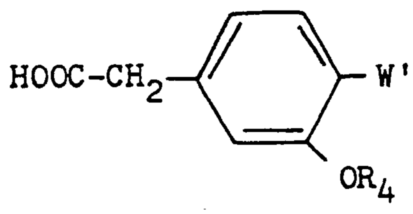

,(III)

wherein

$R_4$ is defined as in claims 1 to 6 and

W' has the meanings given for W in claims 1 to 6, or represents a carboxy group protected by a protecting group, or with the reactive derivatives thereof optionally prepared in the reaction mixture, and if necessary any protecting group used is subsequently split off, or

b) in order to prepare compounds of general formula I wherein W represents a carboxy group:

a compound of general formula

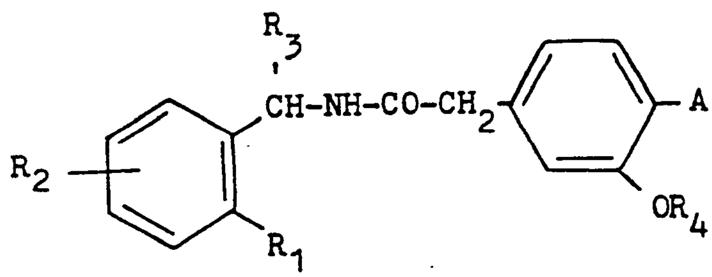

,(IV)

wherein

$R_1$ to $R_4$ are defined as in claims 1 to 6, and

A represents a group which can be converted into a carboxy group by hydrolysis, thermolysis or hydrogenolysis is converted into a corresponding compound by hydrolysis, thermolysis, hydrogenolysis or alcoholysis, or

c) in order to prepare compounds of general formula I wherein $R_4$ represents a hydrogen atom:

a protecting group is split off from a compound of general formula

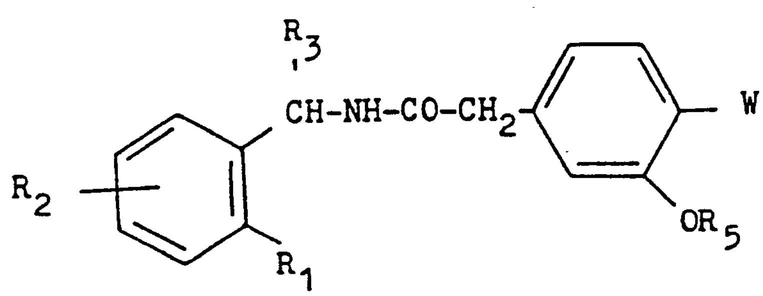

,(V)

wherein

$R_1$ to $R_3$ and W are defined as in claims 1 to 6 and

$R_5$ represents a protecting group for a hydroxy group, or

d) in order to prepare compounds of general formula I wherein $R_4$ represents an alkyl group with 1 to 3 carbon atoms or an allyl group:

a compound of general formula

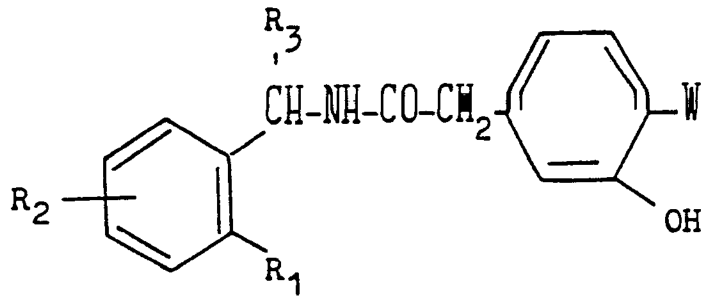

,(VI)

wherein

$R_1$ to $R_3$ and W are defined as in claims 1 to 6, is reacted with a compound of general formula

$$X—R_6 \quad (VII)$$

wherein

$R_6$ represents an alkyl group with 1 to 3 carbon atoms or an allyl group and

X represents a nucleophilically exchangeable group such as a halogen atom, a sulphonyloxy group or, together with the adjacent hydrogen atom, a diazo group, if $R_6$ represents an alkyl group with 1 to 3 carbon atoms, and if necessary the compound thus obtained is subsequently hydrolysed, or

e) in order to prepare compounds of general formula I wherein $R_1$, $R_2$ and $R_3$ are defined as in claims 1 to 6, $R_4$ represents a hydrogen atom or an alkyl group with 1 to 3 carbon atoms and W represents a methyl, formyl, carboxy or alkoxycarbonyl group, wherein the alkoxy part may contain 1 to 4 carbon atoms, a compound of general formula

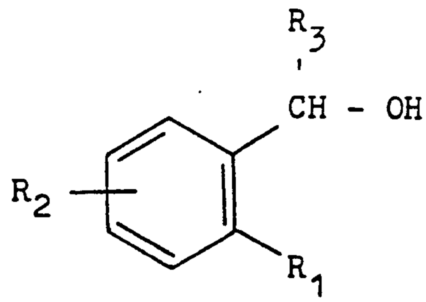

,(VIII)

wherein

$R_1$ to $R_3$ are defined as in claims 1 to 6, is reacted with a compound of general formula

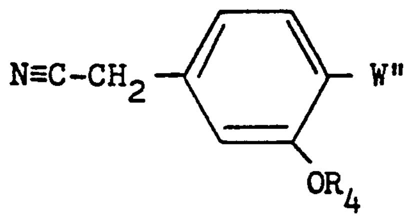

,(IX)

wherein

$R_4$ is defined as in claims 1 to 6 and

W'' represents a methyl, formyl, carboxy or alkoxycarbonyl group, whilst the alkoxy part may contain 1 to 4 carbon atoms, or

f) in order to prepare compounds of general formula I wherein

$R_4$ represents a hydrogen atom or an alkyl group with 1 to 3 carbon atoms,

$R_3$ represents an alkyl group with 1 or 2 carbon atoms substituted by a tetrahydrofuranyl, tetrahydropyranyl or cycloalkyl group, in which the cycloalkyl part may contain 5 to 7 carbon atoms, and

W represents a methyl, hydroxymethyl, carboxy or alkoxycarbonyl group, whilst the alkoxy part may contain from 1 to 4 carbon atoms,

a compound of general formula

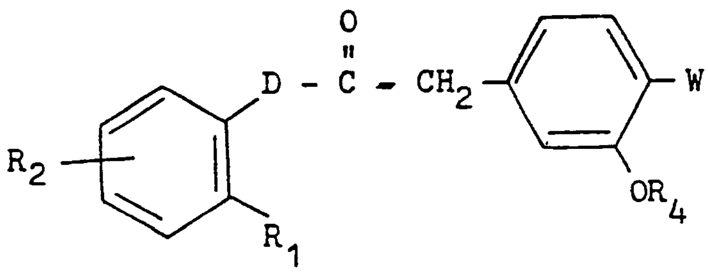

, (X)

wherein

$R_1$, $R_2$, $R_4$ and W are defined as in claims 1 to 6 and

D represents a group of formula

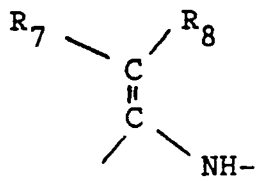

wherein

$R_7$ and $R_8$ together with the carbon atom between them represent an alkylidene group with 1 or 2 carbon atoms substituted by a tetrahydrofuranyl, tetrahydropyranyl or cycloalkyl group, wherein the cycloalkyl part may contain 5 to 7 carbon atoms, is reduced and

subsequently, if desired, a racemic compound of general formula I thus obtained is resolved into the enantiomers thereof by chromatography on chiral phases, and/or

a compound of general formula I thus obtained is converted into the salts thereof, particularly the physiologically acceptable salts thereof with inorganic or organic acids or bases.